# EUROPEAN PATENT SPECIFICATION

(11) **EP 3 793 607 B1**
(45) Date of publication and mention of the grant of the patent: **02.12.2026**
(21) Application number: 19728320.3
(22) Date of filing: 14.05.2019
(51) Int. Cl.: A61K 39/395, A61K 45/06, A61P 35/00

(54) **NKG2A NEUTRALIZING ANTIBODIES, PREFERABLY MONALIZUMAB, IN COMBINATION WITH A PD-1 NEUTRALIZING AGENT TO TREAT CANCER**
NKG2A-NEUTRALISIERENDE ANTIKÖRPER, VORZUGSWEISE MONALIZUMAB, IN KOMBINATION MIT EINEM PD-1-NEUTRALISIERENDEN MITTEL ZUR BEHANDLUNG VON KREBS
ANTICORPS NEUTRALISANTS ANTI-NKG2A, DE PRÉFÉRENCE LE MONALIZUMAB, EN COMBINAISON AVEC UN AGENT NEUTRALISANT PD-1 POUR TRAITER LE CANCER

(30) Priority: 15.05.2018 US 201862671521 P
(43) Date of publication of application: 24.03.2021
(73) Proprietor: MedImmune Limited, Cambridge CB2 0AA (GB); Innate Pharma, 13009 Marseille (FR)
(72) Inventor: ABDULLAH, Shaad Essa, Gaithersburg, MD 20878 (US); GUPTA, Ashok Kumar, Gaithersburg, MD 20878 (US); SONG, Xuyang, Gaithersburg, MD 20878 (US)
(74) Representative: Ipsilon Belgium
(86) International application number: PCT/EP2019/062305
(87) International publication number: WO 2019/219658

(56) References cited:
- WO-A1-2017/106656
- WO-A1-2017/125532
- US-A1- 2017 253 658
- NEIL HOWARD SEGAL ET AL: "First-in-human dose escalation of monalizumab plus durvalumab, with expansion in patients with metastatic microsatellite-stable colorectal cancer. | Journal of Clinical Oncology", 1 June 2018 (2018-06-01), XP055611099, Retrieved from the Internet <URL:https://ascopubs.org/doi/abs/10.1200/JCO.2018.36.15_suppl.3540> [retrieved on 20190806]

## Description

### FIELD OF THE INVENTION

This invention relates to the use of NKG2A-neutralizing antibody and PD-1 neutralizing agent to treat cancer, notably cancer that is not characterized by DNA mismatch repair deficiency. Included is treatment of MSS cancer. The invention is particularly useful for treating advanced recurrent or metastatic colorectal cancer.

### BACKGROUND OF THE INVENTION

NK cell activity is regulated by a complex mechanism that involves both activating and inhibitory signals. Several distinct NK-specific receptors have been identified that play an important role in the NK cell mediated recognition and killing of HLA Class I deficient target cells. Natural Cytotoxicity Receptors (NCR) refers to a class of activating receptor proteins, and the genes expressing them, that are specifically expressed in NK cells. Examples of NCRs include NKp30, NKp44, and NKp46. These receptors are members of the Ig superfamily, and their cross-linking, induced by specific monoclonal antibodies, leads to a strong NK cell activation resulting in increased intracellular Ca⁺⁺ levels, triggering of cytotoxicity, and lymphokine release, and an activation of NK cytotoxicity against many types of target cells.

CD94/NKG2A is an inhibitory receptor found on subsets of lymphocytes. CD94/NKG2A restricts cytokine release and cytotoxic responses of certain lymphocytes towards cells expressing the CD94/NKG2A-ligand HLA-E. HLA-E has also been found to be secreted in soluble form by certain tumor cells and activated endothelial cells. Antibodies that inhibit CD94/NKG2A signalling may increase the cytokine release and cytolytic activity of lymphocytes towards HLA-E positive target cells, such as responses of CD94/NKG2A-positive NK cells towards HLA-E expressing tumor cells or virally infected cells. Neutralizing anti-NKG2A antibodies may therefore be useful in the treatment of cancer.

Colorectal cancer (CRC) accounts for 10%-15% of all cancers and is the leading cause of cancer deaths in the Western world. Standard of care for treatment of metastatic CRC (mCRC) remains the use of cytotoxic agents. More recently, immunotherapeutic agents have been tested in CRC. Le et al. (N Engl J Med. 2015;372:2509-2520) conducted a phase 2 clinical study in CRC with pembrolizumab, an anti-programmed death 1 immune checkpoint inhibitor, finding that the immune-related objective response rate and immune-related PFS rate were 40% (4 of 10 patients) and 78% (7 of 9 patients) for MSI-H CRCs and 0% (0 of 18 patients) and 11% (2 of 18 patients) for microsatellite stable/proficient MSS CRCs, respectively. Only 1 of 10 patients with MSI-H CRC experienced disease progression, as compared to 11/18 MSS CRC patients.

WO2017/125532 relates to NKG2A-neutralizing agents and combinations of NKG2A-neutralizing agents and PD-1 neutralizing agents for the treatment of cancer. WO2017/106656 relates to antibody molecules that specifically bind to PD-1 and treatment or prevention of cancerous or infectious conditions and disorders.

Unfortunately, chemotherapeutic agents and/or targeted therapies do not provide sufficient and/or lasting anti-tumor responses patients having non-MSI-H CRC. There is thus a need in the art for improved benefit to patients treated without DNA repair deficiencies.

### SUMMARY OF THE INVENTION

The invention is set out in the appended claims and relates to a NKG2A neutralizing agent for use in a method of treating a cancer and/or eliciting an anti-tumor immune response in an individual in need thereof, wherein said individual has a tumor that is not MSI-High (MSI-H) and/or not DNA mismatch repair (MMR) defective, the method comprising administering to said individual a therapeutically effective amount of the NKG2A neutralizing agent and a therapeutically effective amount of a PD-1 neutralizing agent, wherein said NKG2A neutralizing agent is an antibody that inhibits binding of NKG2A to HLA-E.

The invention also relates to a PD-1 neutralizing agent for use in a method of treating a cancer and/or eliciting an antitumor immune response in an individual in need thereof, wherein said individual has a tumor that is not MSI-High (MSI-H) and/or not DNA mismatch repair (MMR) defective, the method comprising administering to said individual a therapeutically effective amount of the PD-1 neutralizing agent and a therapeutically effective amount of a NKG2A neutralizing agent, wherein said NKG2A neutralizing agent is an antibody that inhibits binding of NKG2A to HLA-E.

The invention also relates to a kit for use in the treatment of a MSS-CRC in a patient in need thereof, wherein:
i. the kit comprises a pharmaceutical composition containing a NKG2A neutralizing agent such as an anti-NKG2A antibody, and a PD-1 neutralizing agent such as an anti-PD-1 antibody or an anti-PD-L1 antibody; or
ii. the kit comprises a first pharmaceutical composition containing a PD-1 neutralizing agent such as an anti-PD-1 antibody or an anti-PD-L1 antibody, and a second pharmaceutical composition containing a NKG2A neutralizing agent such as an anti-NKG2A antibody; or
iii. the kit comprises a pharmaceutical composition containing a NKG2A neutralizing agent such as an anti-NKG2A antibody, and the treatment comprises administering to said patient a therapeutically effective amount of the NKG2A neutralizing agent and a therapeutically effective amount of a PD-1 neutralizing agent such as an anti-PD-1 antibody or an anti-PD-L1 antibody; or
iv. the kit comprises a pharmaceutical composition containing a PD-1 neutralizing agent such as an anti-PD-1 antibody or an anti-PD-L1 antibody, and the treatment comprises administering to said patient a therapeutically effective amount of the PD-1 neutralizing agent and a therapeutically effective amount of a NKG2A neutralizing agent,
wherein said NKG2A neutralizing agent is an antibody that inhibits binding of NKG2A to HLA-E, optionally wherein said NKG2A neutralizing agent is monalizumab and said PD-1 neutralizing agent is durvalumab.

Any references in this description to methods of treating a disease, are to be interpreted as references to compounds, agents or compositions (as disclosed herein) for use in those methods.

The technical disclosure set out below may in some respects go beyond the scope of the claims. Elements of the disclosure which do not fall within the scope of the claims are provided for information.

The present invention arises, inter alia, from the observation that human patients with MSS-CRC showed promising anti-tumor responses when treated with the NKG2A-neutralizing agent monalizumab and the PD-1-neutralizing agent durvalumab. While PD-1-neutralizing agents had been thought not well suited for MSS-CRC, it has been surprisingly found that the combination of these two agents could be beneficial for treating MSS-CRC. Without willing to be bound by theory, it could be that neutralization of the HLA-E/NKG2A axis could render MSS-CRC tumors sensitive to recognition and/or lysis by the host immune system.

In one aspect, the present invention provides methods of treating and/or eliciting an anti-tumor immune response in an individual in need thereof, wherein said individual has a non-MSI-High tumor, comprising treating said individual with an agent that neutralizes the inhibitory receptor NKG2A and an agent that neutralizes PD-1. In one embodiment, the tumor is MSI-Low. In another embodiment, the tumor is MSS. In one embodiment, the cancer or tumor is colorectal cancer (CRC), optionally advanced recurrent or metastatic colorectal cancer (mCRC).

In one aspect, the present invention provides methods of treating a cancer and/or eliciting an anti-tumor immune response in an individual in need thereof, wherein said individual has a tumor that is not DNA mismatch repair (MMR) defective, comprising treating said individual with an agent that neutralizes the inhibitory receptor NKG2A and an agent that neutralizes PD-1. In one embodiment, the tumor is MSI-Low. In another embodiment, the tumor is MSS. In one embodiment, the cancer or tumor is colorectal cancer (CRC), optionally advanced recurrent or metastatic colorectal cancer (mCRC).

In one aspect, the present invention provides methods of treating a cancer and/or eliciting an anti-tumor immune response in an individual in need thereof, wherein said individual has advanced recurrent or metastatic colorectal cancer, optionally metastatic colorectal cancer, that is not MSI-High (MSI-H) comprising treating said individual with an agent that neutralizes the inhibitory receptor NKG2A and an agent that neutralizes PD-1.

In one aspect, the present invention provides methods of treating a cancer and/or eliciting an anti-tumor immune response in an individual in need thereof, wherein said individual has advanced recurrent or metastatic colorectal cancer, optionally metastatic colorectal cancer, that is microsatellite instability-low (MSI-Low) or microsatellite stable (MSS), comprising treating said individual with an agent that neutralizes the inhibitory receptor NKG2A and an agent that neutralizes PD-1.

In one embodiment, provided is a method for treating a cancer, optionally a CRC or mCRC, in an individual comprising: (i) identifying an individual who has a tumor that is not DNA mismatch-repair defective, and (ii) administering to the individual an effective amount of agent that neutralizes the inhibitory receptor NKG2A and an effective amount of agent that neutralizes PD-1.

In one embodiment, provided is a method for treating a cancer, optionally a CRC or mCRC, in an individual comprising: (i) identifying an individual who has a tumor that is not a MSI-High tumor and (ii) administering to the individual an effective amount of agent that neutralizes the inhibitory receptor NKG2A and an effective amount of agent that neutralizes PD-1.

In one embodiment, provided is a method for treating or preventing a cancer, optionally a CRC or mCRC, in an individual comprising: (i) identifying an individual who has a tumor that is a MSS tumor and (ii) administering to the individual an effective amount of agent that neutralizes the inhibitory receptor NKG2A and an effective amount of agent that neutralizes PD-1.

An aspect useful for understanding the invention relates to a method for determining whether an individual having a cancer, optionally a CRC or mCRC, will derive particular benefit from, be responsive to and/or suitable for treatment with an agent that neutralizes the inhibitory receptor NKG2A and an agent that neutralizes PD-1, the method comprising determining whether said individual has a tumor that is DNA mismatch-repair defective, wherein a determination that the tumor is not DNA mismatch-repair defective indicates that the individual will derive particular benefit from, be responsive to and/or suitable for treatment with an agent that neutralizes the inhibitory receptor NKG2A and an agent that neutralizes a human PD-1 polypeptide.

An aspect useful for understanding the invention relates to a method for determining whether an individual having a cancer, optionally a CRC or mCRC, will derive particular benefit from, be responsive to and/or suitable for treatment with an agent that neutralizes the inhibitory receptor NKG2A and an agent that neutralizes PD-1, the method comprising determining whether said individual has a tumor that lacks microsatellite instability (e.g. the individual has a MSS tumor), wherein a determination that the tumor lacks microsatellite instability (e.g. the individual has a MSS tumor) indicates that the individual will derive particular benefit from, be responsive to and/or suitable for treatment with an agent that neutralizes the inhibitory receptor NKG2A and an agent that neutralizes a human PD-1 polypeptide.

Microsatellite instability is the condition of genetic hypermutability that results from impaired DNA mismatch repair (MMR). The presence of MSI represents phenotypic evidence that MMR is not functioning normally. In most cases, the genetic basis for instability in MSI tumors is an inherited germline alteration in any one or more of the five human MMR genes: MSH2, MLH1, MSH6, PMS2, and PMS1. Thus, microsatellite instability in a tumor can be determined by assessing microsatellite markers and/or MMR genes.

In one embodiment, the step of identifying an individual whose tumor does not have microsatellite instability (e.g. whose tumor is microsatellite stable (MSS)) comprises: (a) obtaining a biological sample from the individual comprising tumor cells (e.g. comprising obtaining a biopsy), (b) determining the microsatellite status of tumor cells within the biological sample. A finding that the individual has a tumor that is not characterized by microsatellite instability (e.g., the tumor is MSS, the tumor is characterized by microsatellite stability) indicates that the individual can be advantageously treated with an agent that neutralizes the inhibitory receptor NKG2A and an agent that neutralizes PD-1.

In one embodiment, the step of determining the microsatellite status of tumor cells comprises detecting an alteration in expression of a DNA mismatch repair (MMR) protein in the tumor cells, optionally wherein the MMR protein is selected from MSH2, MLH1, MSH6, PMS2, and PMS1, optionally wherein the MMR gene or protein is selected from MSH2, MLH1, MSH6 and PMS2. In one embodiment, a finding that the individual has a tumor characterized by decreased or absence of at least one MMR protein indicates that the tumor is characterized by microsatellite instability. An individual whose tumor does not have decreased or absence of at least one MMR protein can be specified as indicating that the tumor is characterized by microsatellite stability (e.g. MSS) and can be advantageously treated with an agent that neutralizes the inhibitory receptor NKG2A and an agent that neutralizes PD-1. Decrease in MMR protein can for example be assessed according to standard methods and guidelines for assessing DNA mismatch repair deficiency. Optionally a decrease in expression of a MMR protein may correspond to a decrease compared to a reference value associated with MSS; optionally a decrease in expression of a MMR protein corresponds to a reference value associated with MSI. Alternatively, the decrease of at least one MMR protein can be evaluated by comparison to a reference value that can be, for instance, the level of the same MMR protein(s) measured in a healthy tissue of the same individual, or the level of the same MMR protein(s) measured in a tumor sample of the same individual analyzed at a different period of time, or the average level of the same MMR protein(s) in biological samples from healthy individuals such as individuals not suffering from cancer.

In one embodiment, the step of determining the microsatellite status of tumor cells comprises detecting alterations in microsatellite markers or microsatellite marker panels. Optionally, alterations are detected in one or more microsatellite markers selected from the group consisting of BAT-25, BAT-26, NR-21, NR-24, MONO27, D5S346, D2S123 and D17S250, more particularly from the group consisting of BAT-25, BAT-26, NR-21, NR-24, and MONO27. An individual whose tumor does not have high-frequency microsatellite instability indicates that the tumor is characterized by microsatellite stability (MSS) or low microsatellite instability (MSI-Low) and can be advantageously treated with an agent that neutralizes the inhibitory receptor NKG2A and an agent that neutralizes PD-1.

The agent that neutralizes the inhibitory receptor NKG2A is an antibody that neutralizes the inhibitory activity of the human NKG2A polypeptide by inhibiting the interaction between human HLA-E and human NKG2A proteins. In one embodiment, the agent that neutralizes the inhibitory receptor NKG2A is an antibody that binds human NKG2A polypeptide and neutralizes the inhibitory activity of the human NKG2A polypeptide, inhibiting the interaction between human HLA-E and human NKG2A proteins. In one embodiment, the agent that neutralizes the inhibitory receptor NKG2A is an antibody that binds the human HLA-E polypeptide and inhibits the interaction between human HLA-E and human NKG2A proteins.

In one embodiment, the agent that neutralizes a human PD-1 polypeptide is an anti-PD-1 or anti-PDL-1 antibody that neutralizes the inhibitory activity of PD-1. The individual can be specified to be a human.

An aspect useful for understanding the invention relates to a method of increasing the activity and/or numbers of tumor-infiltrating CD8+ T cells and/or NK cells in an individual who has a tumor (e.g., a CRC, a mCRC) that is not characterized by defective DNA mismatch repair, the method comprising administering to the individual a therapeutically active amount of a compound that neutralizes the inhibitory receptor NKG2A and a therapeutically active amount of a compound that neutralizes PD-1 polypeptide.

An aspect useful for understanding the invention relates to a method of increasing the activity and/or numbers of tumor-infiltrating CD8+ T cells and/or NK cells in an individual who has a tumor (e.g., a CRC, a mCRC) that is not characterized by microsatellite instability (e.g. a cancer characterized as MSS), the method comprising administering to the individual a therapeutically active amount of a compound that neutralizes the inhibitory receptor NKG2A and a therapeutically active amount of a compound that neutralizes PD-1 polypeptide.

In one embodiment, provided is an anti-NKG2A antibody, for use in the treatment of colorectal cancer (e.g., mCRC) that is not characterized by microsatellite instability (e.g. a cancer characterized as MSS), wherein the agent that neutralizes NKG2A is administered in combination with an agent that neutralizes PD-1, optionally an anti-PD-1 or anti-PD-L1 antibody.

In one embodiment, provided is an agent that neutralizes a human PD-1 polypeptide, optionally an anti-PD-L1 antibody or an anti-PD-1 antibody, for use in the treatment of colorectal cancer (e.g., mCRC) that is not characterized by microsatellite instability (e.g. a cancer characterized as MSS), wherein the agent that neutralizes a human PD-1 polypeptide is administered in combination with an anti-NKG2A antibody.

In one aspect of any embodiment herein, the individual has received prior treatment with radiotherapy, surgery, chemotherapy, and/or treatment with a biological agent.

These aspects are more fully described in, and additional aspects, features, and advantages will be apparent from, the description of the invention provided herein.

### Brief Description of Figures

Figure 1 is a 3D representation of the Best Change in Tumor Size from Baseline (in %, left-hand vertical axis) and Duration of Treatment Response in the Evaluable Population (in weeks, z axis) for each patient (horizontal axis) in the expansion cohort of the MSS-CRC trial.
Figure 2 is a 3D representation of the Best Change in Tumor Size from Baseline (in %, left-hand vertical axis) and Duration of Treatment Response in the Evaluable Population (in weeks, z axis) for each patient (horizontal axis) in the expansion cohort of the MSS-CRC trial on a longer time period compared to Figure 1.

### DETAILED DESCRIPTION

### Definitions

As used in the specification, "a" or "an" may mean one or more. As used in the claim(s), when used in conjunction with the word "comprising", the words "a" or "an" may mean one or more than one. As used herein "another" may mean at least a second or more.

Where "comprising" is used, this can optionally be replaced by "consisting essentially of" or by "consisting of".

NKG2A (OMIM 161555) is a member of the NKG2 group of transcripts (Houchins, et al. (1991) J. Exp. Med. 173:1017-1020). NKG2A is encoded by 7 exons spanning 25 kb, showing some differential splicing. Together with CD94, NKG2A forms the heterodimeric inhibitory receptor CD94/NKG2A, found on the surface of subsets of NK cells, α/β T cells, γ/δ T cells, and NKT cells. Similar to inhibitory KIR receptors, it possesses an ITIM in its cytoplasmic domain. As used herein, "NKG2A" refers to any variant, derivative, or isoform of the NKG2A gene or encoded protein. Human NKG2A comprises 233 amino acids in 3 domains, with a cytoplasmic domain comprising residues 1-70, a transmembrane region comprising residues 71-93, and an extracellular region comprising residues 94-233, of the following sequence:

MDNQGVIYSDLNLPPNPKRQQRKPKGNKSSILATEQEITYAELNLQKASQDFQGNDKTYHC
KDLPSAPEKLIVGILGIICLILMASVVTIVVIPSTLIQRHNNSSLNTRTQKARHCGHCP
EEWITYSNSCYYIGKERRTWEESLLACTSKNSSLLSIDNEEEMKFLSIISPSSWIGVFRNSS
HHPWVTMNGLAFKHEIKDSDNAELNCAVLQVNRLKSAQCGSSIIYHCKHKL (SEQ ID NO:
1).

NKG2C (OMIM 602891) and NKG2E (OMIM 602892) are two other members of the NKG2 group of transcripts (Gilenke, et al. (1998) Immunogenetics 48:163-173). The CD94/NKG2C and CD94/NKG2E receptors are activating receptors found on the surface of subsets of lymphocytes such as NK cells and T-cells.

HLA-E (OMIM 143010) is a nonclassical MHC molecule that is expressed on the cell surface and regulated by the binding of peptides, e.g. such as fragments derived from the signal sequence of other MHC class I molecules. Soluble versions of HLA-E have also been identified. In addition to its T-cell receptor binding properties, HLA-E binds subsets of natural killer (NK) cells, natural killer T-cells (NKT) and T cells (α/β and γ/δ), by binding specifically to CD94/NKG2A, CD94/NKG2B, and CD94/NKG2C (see, e.g., Braud et al. (1998) Nature 391:795-799). Surface expression of HLA-E protects target cells from lysis by CD94/NKG2A+ NK, T, or NKT cell clones. As used herein, "HLA-E" refers to any variant, derivative, or isoform of the HLA-E gene or encoded protein.

In the context of the present invention, "NKG2A-", or "CD94/NKG2A-", "positive lymphocyte", or "restricted lymphocyte", refers to cells of the lymphoid lineage (e.g. NK-, NKT- and T-cells) expressing CD94/NKG2A on the cell-surface, which can be detected by e.g. flow-cytometry using antibodies that specifically recognize a combined epitope on CD94 and NKG2A or and epitope on NKG2A alone. "NKG2A positive lymphocyte" also includes immortal cell lines of lymphoid origin (e.g. NKL, NK-92).

In the context of the present invention, "reduces the inhibitory activity of NKG2A", "neutralizes NKG2A" or "neutralizes the inhibitory activity of NKG2A" refers to a process in which CD94/NKG2A is inhibited in its capacity to negatively affect intracellular processes leading to lymphocyte responses such as cytokine release and cytotoxic responses. This can be measured for example in a NK- or T-cell based cytotoxicity assay, in which the capacity of a therapeutic compound to stimulate killing of HLA-E positive cells by CD94/NKG2A positive lymphocytes is measured. In one embodiment, an antibody preparation that neutralizes NKG2A causes at least a 10% augmentation in the cytotoxicity of a CD94/NKG2A-restricted lymphocyte, optionally at least a 40% or 50% augmentation in said lymphocyte cytotoxicity, optionally at least a 70% augmentation in said lymphocyte cytotoxicity", optionally at least a 70% augmentation of NK cytotoxicity, and referring to the cytotoxicity assays described herewith. If an anti-NKG2A antibody reduces or blocks CD94/NKG2A interactions with HLA-E, it may increase the cytotoxicity of CD94/NKG2A-restricted lymphocytes. This can be evaluated, for example, in a standard 4-hour in vitro cytotoxicity assay using, e.g., NK cells that express CD94/NKG2A, and target cells that express HLA-E. Such NK cells do not efficiently kill targets that express HLA-E because CD94/NKG2A recognizes HLA-E, leading to initiation and propagation of inhibitory signaling that prevents lymphocyte-mediated cytolysis. Such an in vitro cytotoxicity assay can be carried out by standard methods that are well known in the art, as described for example in Coligan et al., eds., Current Protocols in Immunology, Greene Publishing Assoc. and Wiley Interscience, N.Y., (1992, 1993). Chromium release and/or other parameters to assess the ability of the antibody to stimulate lymphocytes to kill target cells such as P815, K562 cells, or appropriate tumor cells are also disclosed in Sivori et al., J. Exp. Med. 1997;186:1129-1136; Vitale et al., J. Exp. Med. 1998; 187:2065-2072; Pessino et al. J. Exp. Med. 1998;188:953-960; Neri et al. Clin. Diag. Lab. Immun. 2001;8:1131-1135; Pende et al. J. Exp. Med. 1999;190:1505-1516. The target cells are labeled with ⁵¹Cr prior to addition of NK cells, and then the killing is estimated as proportional to the release of ⁵¹Cr from the cells to the medium, as a result of killing. The addition of an antibody that prevents CD94/NKG2A from binding to HLA-E results in prevention of the initiation and propagation of inhibitory signaling via CD94/NKG2A. Therefore, addition of such agents results in increases in lymphocyte-mediated killing of the target cells. This step thereby identifies agents that prevent CD94/NKG2A-induced negative signaling by, e.g., blocking ligand binding. In a particular ⁵¹Cr-release cytotoxicity assay, CD94/NKG2A-expressing NK effector-cells can kill HLA-E-negative LCL 721.221 target cells, but less well HLA-E-expressing LCL 721.221-Cw3 control cells. In contrast, YTS effector-cells that lack CD94/NKG2A kill both cell-lines efficiently. Thus, NK effector cells kill less efficiently HLA-E⁺ LCL 721.221-Cw3 cells due to HLA-E-induced inhibitory signaling via CD94/NKG2A. When NK cells are pre-incubated with blocking anti-CD94/NKG2A antibodies described herewith in such a ⁵¹Cr-release cytotoxicity assay, HLA-E-expressing LCL 721.221-Cw3 cells are more efficiently killed, in an antibody-concentration-dependent fashion. The inhibitory activity (i.e. cytotoxicity enhancing potential) of an anti-NKG2A antibody can also be assessed in any of a number of other ways, e.g., by its effect on intracellular free calcium as described, e.g., in Sivori et al., J. Exp. Med. 1997;186:1129-1136. Activation of NK cell cytotoxicity can be assessed for example by measuring an increase in cytokine production (e.g. IFN-γ production) or cytotoxicity markers (e.g. CD107 or CD137 mobilization). In an exemplary protocol, IFN-y production from PBMC is assessed by cell surface and intracytoplasmic staining and analysis by flow cytometry after 4 days in culture. Briefly, Brefeldin A (Sigma Aldrich) is added at a final concentration of 5 µg/ml for the last 4 hours of culture. The cells are then incubated with anti-CD3 and anti-CD56 monoclonal antibody prior to permeabilization (IntraPrep^{™}; Beckman Coulter) and staining with PE-anti-IFN-y or PE-IgG1 (Pharmingen). GM-CSF and IFN-y production from polyclonal activated NK cells are measured in supernatants using ELISA (GM-CSF: DuoSet Elisa, R&D Systems, Minneapolis, MN, IFN-y: OptEIA set, Pharmingen).

An aspect useful for understanding the invention relates to a method of increasing the activity and/or numbers of tumor-infiltrating CD8+ T cells and/or NK cells in an individual who has a tumor (e.g., a CRC, a mCRC) that is not characterized by defective DNA mismatch repair, the method comprising administering to the individual a therapeutically active amount of a compound that neutralizes the inhibitory receptor NKG2A and a therapeutically active amount of a compound that neutralizes PD-1 polypeptide.

An aspect useful for understanding the invention relates to a method of increasing the activity and/or numbers of tumor-infiltrating CD8+ T cells and/or NK cells in an individual who has a tumor (e.g., a CRC, a mCRC) that is not characterized by microsatellite instability (e.g. a cancer characterized as MSS), the method comprising administering to the individual a therapeutically active amount of a compound that neutralizes the inhibitory receptor NKG2A and a therapeutically active amount of a compound that neutralizes PD-1 polypeptide.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it such as a preventive early asymptomatic intervention; (b) inhibiting the disease, e.g., arresting its development; or relieving the disease, e.g., causing regression of the disease and/or its symptoms or conditions such as improvement or remediation of damage, for example in a subject who has been diagnosed as having the disease. Optionally, treatment may cause (e.g. may be characterized as a method of causing) a decrease in tumor burden, a decrease in the size and/or number of lesions, a decrease or delay in the progression of cancer (e.g., an increase in progression-free survival), a delay or prevention of cancer metastasis and/or an increase in survival. Optionally, treatment may cause or provide (e.g. may be characterized as a method of causing or providing) stable disease, a partial response or a complete response in a subject, e.g. according to standard criteria, optionally RECIST criteria.

The term "biopsy" as used herein is defined as removal of a tissue for the purpose of examination, such as to establish diagnosis. Examples of types of biopsies include by application of suction, such as through a needle attached to a syringe; by instrumental removal of a fragment of tissue; by removal with appropriate instruments through an endoscope; by surgical excision, such as of the whole lesion; and the like.

The term "antibody," as used herein, refers to polyclonal and monoclonal antibodies. Depending on the type of constant domain in the heavy chains, antibodies are assigned to one of five major classes: IgA, IgD, IgE, IgG, and IgM. Several of these are further divided into subclasses or isotypes, such as IgG1, IgG2, IgG3, IgG4, and the like. An exemplary immunoglobulin (antibody) structural unit comprises a tetramer. Each tetramer is composed of two identical pairs of polypeptide chains, each pair having one "light" (about 25 kDa) and one "heavy" chain (about 50-70 kDa). The N-terminus of each chain defines a variable region of about 100 to 110 or more amino acids that is primarily responsible for antigen recognition. The terms variable light chain (V_{L}) and variable heavy chain (V_{H}) refer to these light and heavy chains respectively. The heavy-chain constant domains that correspond to the different classes of immunoglobulins are termed "alpha," "delta," "epsilon," "gamma" and "mu," respectively. The subunit structures and three-dimensional configurations of different classes of immunoglobulins are well known. IgG are the exemplary classes of antibodies employed herein because they are the most common antibodies in the physiological situation and because they are most easily made in a laboratory setting. Optionally the antibody is a monoclonal antibody. Particular examples of antibodies are humanized, chimeric, human, or otherwise-human-suitable antibodies. "Antibodies" also includes any fragment or derivative of any of the herein described antibodies.

The term "specifically binds to" means that an antibody can bind preferably in a competitive binding assay to the binding partner, e.g. NKG2A for an anti-NKG2A antibody, PD-L1 for an anti-PD-L1 antibody, PD-1 for an anti-PD-1 antibody, as assessed using either recombinant forms of the proteins, epitopes therein, or native proteins present on the surface of isolated target cells. Competitive binding assays and other methods for determining specific binding are well known in the art. For example binding can be detected via radiolabels, physical methods such as mass spectrometry, or direct or indirect fluorescent labels detected using, e.g., cytofluorometric analysis (e.g. FACScan). Binding above the amount seen with a control, non-specific agent indicates that the agent binds to the target. An agent that specifically binds NKG2A may bind NKG2A alone or NKG2A as a dimer with CD94.

When an antibody is said to "compete with" a particular monoclonal antibody, it means that the antibody competes with the monoclonal antibody in a binding assay using either recombinant molecules (e.g., NKG2A) or surface expressed molecules (e.g., NKG2A). For example, if a test antibody reduces the binding of an antibody having a heavy chain variable region of any of SEQ ID NOS: 4-8 and a light chain variable region of SEQ ID NO: 9 to a NKG2A polypeptide or NKG2A-expressing cell in a binding assay, the antibody is said to "compete" respectively with such antibody.

The term "affinity", as used herein, means the strength of the binding of an antibody to an epitope. The affinity of an antibody is given by the dissociation constant Kd, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Kₐ is defined by 1/Kd. Methods for determining the affinity of monoclonal antibodies can be found in Harlow, et al., Antibodies: A Laboratory Manual, Cold Spring Harbor Laboratory Press, Cold Spring Harbor, N.Y., 1988), Coligan et al., eds., Current Protocols in Immunology, Greene Publishing Assoc. and Wiley Interscience, N.Y., (1992, 1993), and Muller, Meth. Enzymol. 92:589-601 (1983). One standard method well known in the art for determining the affinity of monoclonal antibodies is the use of surface plasmon resonance (SPR) screening (such as by analysis with a BIAcore^{™} SPR analytical device).

Within the context herein a "determinant" designates a site of interaction or binding on a polypeptide.

The term "epitope" refers to an antigenic determinant, and is the area or region on an antigen to which an antibody binds. A protein epitope may comprise amino acid residues directly involved in the binding as well as amino acid residues which are effectively blocked by the specific antigen binding antibody or peptide, *i.e.,* amino acid residues within the "footprint" of the antibody. It is the simplest form or smallest structural area on a complex antigen molecule that can combine with e.g., an antibody or a receptor. Epitopes can be linear or conformational/structural. The term "linear epitope" is defined as an epitope composed of amino acid residues that are contiguous on the linear sequence of amino acids (primary structure). The term "conformational or structural epitope" is defined as an epitope composed of amino acid residues that are not all contiguous and thus represent separated parts of the linear sequence of amino acids that are brought into proximity to one another by folding of the molecule (secondary, tertiary and/or quaternary structures). A conformational epitope is dependent on the 3-dimensional structure. The term 'conformational' is therefore often used interchangeably with 'structural'.

The term "agent" is used herein to denote a chemical compound, a mixture of chemical compounds, a biological macromolecule, or an extract made from biological materials. The term "therapeutic agent" refers to an agent that has biological activity.

For the purposes herein, a "humanized" or "human" antibody refers to an antibody in which the constant and variable framework region of one or more human immunoglobulins is fused with the binding region, e.g. the CDR, of an animal immunoglobulin. Such antibodies are designed to maintain the binding specificity of the non-human antibody from which the binding regions are derived, but to avoid an immune reaction against the non-human antibody. Such antibodies can be obtained from transgenic mice or other animals that have been "engineered" to produce specific human antibodies in response to antigenic challenge (see, e.g., Green et al. (1994) Nature Genet 7:13; Lonberg et al. (1994) Nature 368:856; Taylor et al. (1994) Int Immun 6:579). A fully human antibody also can be constructed by genetic or chromosomal transfection methods, as well as phage display technology, all of which are known in the art (see, e.g., McCafferty et al. (1990) Nature 348:552-553). Human antibodies may also be generated by in vitro activated B cells (see, e.g., U.S. Pat. Nos. 5,567,610 and 5,229,275).

A "chimeric antibody" is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity.

The terms "Fc domain," "Fc portion," and "Fc region" refer to a C-terminal fragment of an antibody heavy chain, e.g., from about amino acid (aa) 230 to about aa 450 of human γ (gamma) heavy chain or its counterpart sequence in other types of antibody heavy chains (e.g., α, δ, ε and µ for human antibodies), or a naturally occurring allotype thereof. Unless otherwise specified, the commonly accepted Kabat amino acid numbering for immunoglobulins is used throughout this disclosure (see Kabat et al. (1991 ) Sequences of Protein of Immunological Interest, 5th ed., United States Public Health Service, National Institute of Health, Bethesda, MD).

The terms "isolated", "purified" or "biologically pure" refer to material that is substantially or essentially free from components which normally accompany it as found in its native state. Purity and homogeneity are typically determined using analytical chemistry techniques such as polyacrylamide gel electrophoresis or high performance liquid chromatography. A protein that is the predominant species present in a preparation is substantially purified.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymer.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, for example, recombinant cells express genes that are not found within the native (nonrecombinant) form of the cell or express native genes that are otherwise abnormally expressed, under expressed or not expressed at all.

Within the context herein, the term antibody that "binds" a polypeptide or epitope designates an antibody that binds said determinant with specificity and/or affinity.

The term "identity" or "identical", when used in a relationship between the sequences of two or more polypeptides, refers to the degree of sequence relatedness between polypeptides, as determined by the number of matches between strings of two or more amino acid residues. "Identity" measures the percent of identical matches between the smaller of two or more sequences with gap alignments (if any) addressed by a particular mathematical model or computer program (i.e., "algorithms"). Identity of related polypeptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988).

Methods for determining identity are designed to give the largest match between the sequences tested. Methods of determining identity are described in publicly available computer programs. Computer program methods for determining identity between two sequences include the GCG program package, including GAP (Devereux et al., Nucl. Acid. Res. 12, 387 (1984); Genetics Computer Group, University of Wisconsin, Madison, Wis.), BLASTP, BLASTN, and FASTA (Altschul et al., J. Mol. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul *et al.,* supra). The well-known Smith Waterman algorithm may also be used to determine identity.

### NKG2A-neutralizing therapeutic agents

The NKG2A neutralizing agent binds an extra-cellular portion of human CD94/NKG2A receptor or its ligand HLA-E and reduces the inhibitory activity of human CD94/NKG2A receptor expressed on the surface of a CD94/NKG2A positive lymphocyte. The agent competes with HLA-E in binding to CD94/NKG2A, i.e. the agent blocks the interaction between CD94/NKG2A and its ligand HLA-E. In another example useful for understanding the invention the agent binds NKG2A but does not compete with HLA-E in binding to CD94/NKG2A; i.e. the agent is capable of binding CD94/NKG2A simultaneously with HLA-E. In one embodiment, the agent is an antibody that binds to NKG2A. The antibody may bind a combined epitope on CD94 and NKG2A or an epitope on NKG2A alone. In another embodiment, the agent is an antibody that binds to HLA-E and inhibits the interaction between human HLA-E and human NKG2A proteins.

In one aspect the NKG2A neutralizing agent is an antibody selected from a fully human antibody, a humanized antibody, and a chimeric antibody. In one aspect, the agent comprises a constant domain derived from a human IgG1, IgG2, IgG3 or IgG4 antibody. In one aspect, the agent is a fragment of an antibody selected from IgA, an IgD, an IgG, an IgE and an IgM antibody. In one aspect, the agent is an antibody fragment selected from a Fab fragment, a Fab' fragment, a Fab'-SH fragment, a F(ab)2 fragment, a F(ab')2 fragment, an Fv fragment, a Heavy chain Ig (a llama or camel Ig), a V_{HH} fragment, a single domain FV, and a single-chain antibody fragment. In one aspect, the agent is a synthetic or semisynthetic antibody-derived molecule selected from a scFV, a dsFV, a minibody, a diabody, a triabody, a kappa body, an IgNAR, and a multispecific antibody.

Optionally, the anti-NKG2A antibodies do not demonstrate substantial specific binding to human Fcy receptors, e.g. CD16. Optionally, the anti-NKG2A antibodies lack substantial specific binding or have low or decreased specific binding to one or more, or all of, human CD16, CD32A, CD32B or CD64.Exemplary antibodies may comprise constant regions of various heavy chains that are known not to bind or to have low binding to Fcy receptors. One such example is a human IgG4 constant region. In one embodiment, the IgG4 antibody comprises a modification to prevent the formation of half antibodies (fab arm exchange) in vivo, e.g., the antibody comprises an IgG4 heavy chain comprising a serine to proline mutation in residue 241, corresponding to position 228 according to the EU-index (Kabat et al., "Sequences of proteins of immunological interest", 5th ed., NIH, Bethesda, ML, 1991). Such modified IgG4 antibodies will remain intact in vivo and maintain a bivalent (high affinity) binding to NKG2A, as opposed to native IgG4 that will undergo fab arm exchange in vivo such that they bind to NKG2A in monovalent manner which can alter binding affinity. Alternatively, antibody fragments that do not comprise constant regions, such as Fab or F(ab')2 fragments, can be used to avoid Fc receptor binding. Fc receptor binding can be assessed according to methods known in the art, including for example testing binding of an antibody to Fc receptor protein in a BIACORE assay. Also, any human antibody type (e.g. IgG1, IgG2, IgG3 or IgG4) can be used in which the Fc portion is modified to minimize or eliminate binding to Fc receptors (see, e.g., WO03101485). Assays such as, e.g., cell based assays, to assess Fc receptor binding are well known in the art, and are described in, e.g., WO03101485.

The present invention thus concerns antibodies binding to NKG2A. In one aspect, the antibody binds to NKG2A with a KD at least 100-fold lower than to human NKG2C and/or NKG2E.

The agent reduces CD94/NKG2A-mediated inhibition of a CD94/NKG2A-expressing lymphocyte by interfering with CD94/NKG2A signalling by interfering with the binding of HLA-E by NKG2A, and optionally by preventing or inducing conformational changes in the CD94/NKG2A receptor, and/or affecting dimerization and/or clustering of the CD94/NKG2A receptor.

In one aspect of the invention, the agent binds to an extracellular portion of NKG2A with a KD at least 100 fold lower than to NKG2C. In a further preferred aspect, the agent binds to an extracellular portion of NKG2A with a KD at least 150, 200, 300, 400, or 10,000 fold lower than to NKG2C. In another aspect of the invention, the agent binds to an extracellular portion of NKG2A with a KD at least 100 fold lower than to NKG2C, NKG2E and/or NKG2H molecules. In a further preferred aspect, the agent binds to an extracellular portion of NKG2A with a KD at least 150, 200, 300, 400, or 10,000 fold lower than to NKG2C, NKG2C and/or NKG2H molecules. This can be measured, for instance, in BiaCore experiments, in which the capacity of agents to bind the extracellular portion of immobilized CD94/NKG2A (e.g. purified from CD94/NKG2 expressing cells, or produced in a bio-system) is measured and compared to the binding of agents to similarly produced CD94/NKG2C and/or other CD94/NKG2 variants in the same assay. Alternatively, the binding of agents to cells that either naturally express, or over-express (e.g. after transient or stable transfection), CD94/NKG2A can be measured and compared to binding of cells expressing CD94/NKG2C and/or other CD94/NKG2 variants. Anti-NKG2A antibodies may optionally bind NKG2B, which is an NKG2A splice variant forming an inhibitory receptor together with CD94. In one embodiment, affinity can be measured using the methods disclosed in U.S. Patent No 8,206,709, for example by assessing binding to covalently immobilized NKG2A-CD94-Fc fusion protein by Biacore as shown in Example 8 of U.S. Patent No 8,206,709.

The anti-NKG2A antibody can be a humanized antibody, for example comprising a VH human acceptor framework from a human acceptor sequence selected from, e.g., VH1_18, VH5_a, VH5_51, VH1_f, and VH1_46, and a JH6 J-segment, or other human germline VH framework sequences known in the art. The VL region human acceptor sequence may be, e.g., VKI_O2/JK4.

In one embodiment, the antibody is a humanized antibody based on antibody Z270. Different humanized Z270 heavy chain variable regions are shown in SEQ ID NOS: 4-8, with optionally further comprising a C-terminal serine (S) residue. The HumZ270VH6 variable region of SEQ ID NO: 4 is based on a human VH5_51 gene; the HumZ270VH1 variable region of SEQ ID NO: 5 is based on a human VH1_18 gene; the humZ270VH5 variable region of SEQ ID NO: 6 is based on a human VH5_a gene; the humZ270VH7 variable region of SEQ ID NO: 7 is based on a human VH1_f gene; and the humZ270VH8 variable region of SEQ ID NO: 8 is based on a human VH1_46 gene; all with a human JH6 J-segment. Each of these antibodies retains high affinity binding to NKG2A, with low likelihood of a host immune response against the antibody as the 6 C-terminal amino acid residues of the Kabat H-CDR2 of each of the humanized constructs are identical to the human acceptor framework. Using the alignment program VectorNTI, the following sequence identities between humZ270VH1 and humZ270VH5, -6, -7, and -8 were obtained: 78,2% (VH1 vs. VH5), 79,0% (VH1 vs. VH6), 88,7% (VH1 vs. VH7), and 96,0% (VH1 vs. VH8).

In one aspect, the agent comprises (i) a heavy chain variable region of SEQ ID NOS: 4-8, or an amino acid sequence at least 50%, 60%, 70%, 80%, 90%, 95%, 98% or 99% identical thereto, and (ii) a light chain variable region of SEQ ID NO: 9, or an amino acid sequence at least 50%, 60%, 70%, 80%, 90%, 95%, 98% or 99% identical thereto. In one aspect, the agent comprises (i) a heavy chain comprising the amino acid sequence of any of SEQ ID NOS: 10-14, or an amino acid sequence at least 50%, 60%, 70%, 80%, 90%, 95%, 98% or 99% identical thereto, and (ii) a light chain comprising the amino acid sequence of SEQ ID NO: 15, or an amino acid sequence at least 50%, 60%, 70%, 80%, 90%, 95%, 98% or 99% identical thereto.

The antibody having a heavy chain variable region of any of SEQ ID NOS: 4-8 and a light chain variable region of SEQ ID NO: 9 neutralizes the inhibitory activity of NKG2A, but does not substantially bind the activating receptors NKG2C, NKG2E or NKG2H. This antibody furthermore competes with HLA-E for binding to NKG2A on the surface of a cell. In one aspect, the agent comprises H-CDR1, H-CDR2 and/or H-CDR3 sequences derived from the heavy chain variable region having the amino acid sequence of any of SEQ ID NOS: 4-8. In one aspect of the invention, the agent comprises L-CDR1, L-CDR2 and/or L-CDR3 sequences derived from the light chain variable region having the amino acid sequence of SEQ ID NO: 9.

### Heavy chain variable regions

**VH6** 
EVQLVQSGAEVKKPGESLKISCKGSGYSFTSYWMNWVRQMPGKGLEWMGRIDPYD
SETHYSPSFQGQVTISADKSISTAYLQWSSLKASDTAMYYCARGGYDFDVGTLY
WFFDVWGQGTTVTVS
(SEQ ID NO: 4)
VH1: 
QVQLVQSGAEVKKPGASVKVSCKASGYTFTSYWMNWVRQAPGQGLEWMGRIDPYDSETHYA
QKLQGRVTMTTDTSTSTAYMELRSLRSDDTAVYYCARGGYDFDVGTLYWFFDVWGQGTTVTVS
(SEQ ID NO: 5)
VH5: 
EVQLVQSGAEVKKPGESLRISCKGSGYSFTSYWMNWVRQMPGKGLEWMGRIDPYD
SETHYSPSFQGHVTISADKSISTAYLQWSSLKASDTAMYYCARGGYDFDVGTLY
WFFDVWGQGTTVTVS (SEQ ID NO: 6)
VH7: 
EVQLVQSGAEVKKPGATVKISCKVSGYTFTSYWMNWVQQAPGKGLEWMGRIDPYDSETHY
AEKFQGRVTITADTSTDTAYMELSSLRSEDTAVYYCATGGYDFDVGTLYWFFDVWGQGTTVTVS
(SEQ ID NO: 7)
VH8: 
QVQLVQSGAEVKKPGASVKVSCKASGYTFTSYWMNWVRQAPGQGLEWMGRIDPYDSETHY
AQKFQGRVTMTRDTSTSTVYMELSSLRSEDTAVYYCARGGYDFDVGTLYWFFDVWGQGTTVTVS
(SEQ ID NO: 8)
Light chain variable region 
DIQMTQSPSSLSASVGDRVTITCRASENIYSYLAWYQQKPGKAPKLLIYNAKTLAEGVPSRFSGS
GSGTDFTLTISSLQPEDFATYYCQHHYGTPRTFGGGTKVEIK (SEQ ID NO: 9)
Heavy Chains (variable region domain amino acids underlined)
**VH6:** 
EVQLVQSGAEVKKPGESLKISCKGSGYSFTSYWMNWVRQMPGKGLEWMGRIDPYD
SETHYSPSFQGQVTISADKSISTAYLQWSSLKASDTAMYYCARGGYDFDVGTLY
WFFDVWGQGTTVTVSSASTKGPSVFPLAPCSRSTSESTAALGCLVKDYFPEPVTVSWNSGALTSGV
HTFPAVLQSSGLYSLSSVVTVPSSSLGTKTYTCNVDHKP
SNTKVDKRVESKYGPPCPPCPAPEFLGGPSVFLFPPKPKDTLMIS
RTPEVTCVVVDVSQEDPEVQFNWYVDGVEVHNAK
TKPREEQFNSTYRVVSVLTVLHQDWLNGKEYKCKVSNKGLPSSIEK
TISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSLGK
(SEQ ID NO: 10)
VH1: 
QVQLVQSGAEVKKPGASVKVSCKASGYTFTSYWMNWVRQAPGQGLEWMGRIDPYDSETHYA
QKLQGRVTMTTDTSTSTAYMELRSLRSDDTAVYYCARGGYDFDVGTLYWFFDVWGQGTTVTVS
SASTKGPSVFPLAPCSRSTSESTAALGCLVKDYFPEPVTVSWNSGALTSGVHTFPAVLQSSGLYS
LSSVVTVPSSSLGTKTYTCNVDHKPSNTKVDKRVESKYGPPCPPCPAPEFLGGPSVFLFPPKPKD
TLMISRTPEVTCVVVDVSQEDPEVQFNWYVDGVEVHNAKTKPREEQFNSTYRVVSVLTVLHQDW
LNGKEYKCKVSNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEALHNHYTQKSLS
LSLGK (SEQ ID NO: 11)
VH5: EVQLVQSGAEVKKPGESLRISCKGSGYSFTSYWMNWVRQMPGKGLEWMGRIDPYD 
SETHYSPSFQGHVTISADKSISTAYLQWSSLKASDTAMYYCARGGYDFDVGTLY
WFFDVWGQGTTVTVSSASTKGPSVFPLAPCSRSTSESTAALGCLVKDYFPEPVTVSWNSGALTSGV
HTFPAVLQSSGLYSLSSVVTVPSSSLGTKTYTCNVDHKP
SNTKVDKRVESKYGPPCPPCPAPEFLGGPSVFLFPPKPKDTLMIS
RTPEVTCVVVDVSQEDPEVQFNWYVDGVEVHNAKTKPREEQFNSTYRVVSVLTVLHQDW
LNGKEYKCKVSNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEALHNHYTQKSLS
LSLGK (SEQ ID NO: 12)
VH7: 
EVQLVQSGAEVKKPGATVKISCKVSGYTFTSYWMNWVQQAPGKGLEWMGRIDPYDSETHY
AEKFQGRVTITADTSTDTAYMELSSLRSEDTAVYYCATGGYDFDVGTLY
WFFDVWGQGTTVTVSSASTKGPSVFPLAPCSRSTSESTAALGCLVKDYFPEPVTVSWNSGALTSGV
HTFPAVLQSSGLYSLSSVVTVPSSSLGTKTYTCNVDHKP
SNTKVDKRVESKYGPPCPPCPAPEFLGGPSVFLFPPKPKDTLMIS
RTPEVTCVVVDVSQEDPEVQFNWYVDGVEVHNAKTKPREEQFNSTYRVVSVLTVLHQDW
LNGKEYKCKVSNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEALHNHYTQKSLS
LSLGK (SEQ ID NO: 13)
VH8: 
QVQLVQSGAEVKKPGASVKVSCKASGYTFTSYWMNWVRQAPGQGLEWMGRIDPYDSETHY
AQKFQGRVTMTRDTSTSTVYMELSSLRSEDTAVYYCARGGYDFDVGTLYWFFDVWGQGTTVTVS
SASTKGPSVFPLAPCSRSTSESTAALGCLVKDYFPEPVTVSWNSGALTSGVHT
FPAVLQSSGLYSLSSVVTVPSSSLGTKTYTCNVDHKPSNTKVDKRVESKYGPPCPPCPAPE
FLGGPSVFLFPPKPKDTLMISRTPEVTCVVVDVSQEDPEVQFNWYVDGVEVHNAK
TKPREEQFNSTYRVVSVLTVLHQDWLNGKEYKCKVSNKGLPSSIEK
TISKAKGQPREPQVYTLPPSQEEMTKNQVSLTCLVKGFYPSDIAVEWESNGQPEN
NYKTTPPVLDSDGSFFLYSRLTVDKSRWQEGNVFSCSVMHEALHNHYTQKSLSLSLGK (SEQ ID NO:
14)
**Light chain** (variable region domain amino acids underlined) 
DIQMTQSPSSLSASVGDRVTITCRASENIYSYLAWYQQKPGKAPKLLIYNAKTLAEGVPSRFSGS
GSGTDFTLTISSLQPEDFATYYCQHHYGTPRTFGGGTKVEIKRTVAAPSVFIFPPSDEQLKSGTASVVC
LLNNFYPREAKVQWKVDNALQSGNSQESVTEQDSKDSTYSLSSTLTLSKADYEKHKVYACEVTHQG
LSSPVTKSFNRGEC (SEQ ID NO: 15)
**Monalizumab, CDRs on heavy and light chains**
Heavy chain CDRs, according to Kabat numbering scheme:
   H-CDR1: SYWMN (SEQ ID NO: 16)
   H-CDR2: RIDPYDSETHYAQKLQG (SEQ ID NO: 17)
   H-CDR3: GGYDFDVGTLYWFFDV (SEQ ID NO: 18)
Light chain CDRs according to Kabat numbering scheme:
   L-CDR1: RASENIYSYLA (SEQ ID NO: 19)
   L-CDR2: NAKTLAE (SEQ ID NO: 20)
   L-CDR3: QHHYGTPRT (SEQ ID NO: 21)

In one aspect, the anti-NKG2A antibody is an antibody comprising a H-CDR1 corresponding to residues 31-35 of SEQ ID NOS: 4-8 (or of SEQ ID NOS: 10-14), a H-CDR2 corresponding to residues 50-60 (optionally 50-66 when including amino acids of human origin) of SEQ ID NOS: 4-8 (or of SEQ ID NOS: 10-14), and a H-CDR3 corresponding to residues 99-114 (95-102 according to Kabat) of SEQ ID NOS: 4-8 (or of SEQ ID NOS: 10-14). In one embodiment, the H-CDR2 corresponding to residues 50-66 of SEQ ID NOS: 4-8 (or of SEQ ID NOS: 10-14). Optionally, a CDR may comprise one, two, three, four, or more amino acid substitutions.

In one aspect, the anti-NKG2A antibody is an antibody comprising a L-CDR1 corresponding to residues 24-34 of SEQ ID NOS: 9 or 15, a L-CDR2 corresponding to residues 50-56 of SEQ ID NOS: 9 or 15, and an L-CDR3 corresponding to residues 89-97 of SEQ ID NOS: 9 or 15. Optionally, a CDR may comprise one, two, three, four, or more amino acid substitutions.

In one aspect, the anti-NKG2A antibody is an antibody comprising a H-CDR1 corresponding to residues 31-35 of SEQ ID NOS: 4-8, a H-CDR2 corresponding to residues 50-60 (optionally 50-66) of SEQ ID NOS: 4-8, and a H-CDR3 corresponding to residues 99-114 (95-102 according to Kabat) of SEQ ID NOS: 4-8, a L-CDR1 corresponding to residues 24-34 of SEQ ID NO: 9, a L-CDR2 corresponding to residues 50-56 of SEQ ID NO: 9, and an L-CDR3 corresponding to residues 89-97 of SEQ ID NO: 9.

In one aspect, the anti-NKG2A antibody is an antibody comprising the heavy chain H-CDR1, H-CDR2 and H-CDR3 domains having the amino acid sequences of SEQ ID NOS: 16-18, and the light chain L-CDR1, L-CDR2 and L-CDR3 domains having the amino acid sequences of SEQ ID NOS: 19-21, respectively.

In one aspect, the agent is monalizumab, an anti-NKG2A antibody having the heavy chain variable region amino acid sequence of SEQ ID NO: 5 and the light chain variable region amino acid sequence of SEQ ID NO: 9. In one aspect, the agent is monalizumab, an anti-NKG2A antibody having the heavy chain amino acid sequence of SEQ ID NO: 11 and the light chain amino acid sequence of SEQ ID NO: 15.

In one aspect, the agent comprises H-CDR1, H-CDR2 and/or H-CDR3 sequences derived from the VH having the amino acid sequence of SEQ ID NO: 22. In one aspect of the invention, the agent comprises L-CDR1, L-CDR2 and/or L-CDR3 sequences derived from the VL having the amino acid sequence of SEQ ID NO: 23. In one aspect, the agent comprises H-CDR1, H-CDR2 and/or H-CDR3 sequences derived from the VH having the amino acid sequence of SEQ ID NO: 22, and L-CDR1, L-CDR2 and/or L-CDR3 sequences derived from the VL having the amino acid sequence of SEQ ID NO: 23. The antibody having the heavy chain variable region of SEQ ID NO: 22 and a light chain variable region of SEQ ID NO: 23 neutralizes the inhibitory activity of NKG2A, and also binds the activating receptors NKG2C, NKG2E or NKG2H. This antibody does not compete with HLA-E for binding to NKG2A on the surface of a cell (i.e. it is a non-competitive antagonist of NKG2A).

EVQLVESGGGLVKPGGSLKLSCAASGFTFSSYAMSWVRQSPEKRLEWVAEISSGGSYTYY
PDTVTGRFTISRDNAKNTLYLEISSLRSEDTAMYYCTRHGDYPRFFDVWGAGTTVTVSS
(SEQ ID NO: 22)

QIVLTQSPALMSASPGEKVTMTCSASSSVSYIYWYQQKPRSSPKPWIYLTSNLASGVPAR
FSGSGSGTSYSLTISSMEAEDAATYYCQQWSGNPYTFGGGTKLEIK
(SEQ ID NO: 23)

In one aspect, the agent comprises amino acid residues 31-35, 50-60, 62, 64, 66, and 99-108 of the variable-heavy (V_{H}) domain (SEQ ID NO: 22 and amino acid residues 24-33, 49-55, and 88-96 of the variable-light (V_{L}) domain (SEQ ID NO: 23), optionally with one, two, three, four, or more amino acid substitutions. In one aspect, the agent is a humanized antibody, for example an agent comprising heavy and light chain variable regions as disclosed in PCT publication no. WO2009/092805.

In one aspect, the agent is a fully human antibody which has been raised against the CD94/NKG2A epitope to which any of the aforementioned antibodies bind.

It will be appreciated that, while the aforementioned antibodies can be used, other antibodies can recognize and be raised against any part of the NKG2A polypeptide so long as the antibody causes the neutralization of the inhibitory activity of NKG2A. For example, any fragment of NKG2A, preferably but not exclusively human NKG2A, or any combination of NKG2A fragments, can be used as immunogens to raise antibodies, and the antibodies can recognize epitopes at any location within the NKG2A polypeptide, so long as they can do so on NKG2A expressing NK cells as described herein. Optionally, the epitope is the epitope specifically recognized by an antibody having a heavy chain variable region of SEQ ID NOS: 4-8 and a light chain variable region of SEQ ID NO: 9.

In one aspect, the agent competes with humZ270 antibody disclosed in U.S. Patent No 8,206,709 in binding to the extra-cellular portion of human CD94/NKG2A receptor. Competitive binding can be measured, for instance, in BiaCore experiments, in which the capacity of agents is measured, for binding the extracellular portion of immobilized CD94/NKG2A receptor (e.g. purified from CD94/NKG2 expressing cells, or produced in a bio-system) saturated with humZ270. Alternatively, the binding of agents to cells is measured that either naturally express, or over-express (e.g. after transient or stable transfection), CD94/NKG2A receptor, and which have been pre-incubated with saturating doses of Z270. In one embodiment, competitive binding can be measured using the methods disclosed in U.S. Patent No 8,206,709, for example by assessing binding to Ba/F3-CD94-NKG2A cells by flow cytometry as shown in Example 15 of U.S. Patent No 8,206,709.

### PD-1 neutralizing agents

As used herein, the terms "PD-1" refers to the protein Programmed Death 1 (PD-1) (also referred to as "Programmed Cell Death 1"), an inhibitory member of the CD28 family of receptors, that also includes CD28, CTLA-4, ICOS and BTLA. The complete human PD-1 sequence can be found under GenBank Accession No. U64863, shown as follows:

MQIPQAPWPVVWAVLQLGWRPGWFLDSPDRPWNPPTFFPALLVVTEGDNATFTCSFSNTSES
FVLNWYRMSPSNQTDKLAAFPEDRSQPGQDCRFRVTQLPNGRDFHMSVVRARRNDSGTYLCG
AISLAPKAQIKESLRAELRVTERRAEVPTAHPSPSPRPAGQFQTLVVGVVGGLLGSLVLLVW
VLAVICSRAARGTIGARRTGQPLKEDPSAVPVFSVDYGELDFQWREKTPEPPVPCVPEQTEY
ATIVFPSGMGTSSPARRGSADGPRSAQPLRPEDGHCSWPL (SEQ ID NO: 2).

"PD-1" also includes any variant, derivative, or isoform of the PD-1 gene or encoded protein. PD-1 is expressed on activated B cells, T cells, and myeloid cells (Okazaki et al. (2002) Curr. Opin. Immunol. 14: 391779-82; Bennett et al. (2003) J Immunol 170:711-8). The initial members of the family, CD28 and ICOS, were discovered by functional effects on augmenting T cell proliferation following the addition of monoclonal antibodies (Hutloff et al. (1999) Nature 397:263-266; Hansen et al. (1980) Immunogenics 10:247-260). Two ligands for PD-1 have been identified, PD-L1 and PD-L2, that have been shown to downregulate T cell activation upon binding to PD-1 (Freeman et al. (2000) J Exp Med 192:1027-34; Latchman et al. (2001) Nat Immunol 2:261-8; Carter et al. (2002) Eur J Immunol 32:634-43). Both PD-L1 and PD-L2 are B7 homologs that bind to PD-1, but do not bind to other CD28 family members.

The complete human PD-L1 sequence can be found under UniProtKB/Swiss-Prot, identifier Q9NZQ7-1, shown as follows:

MRIFAVFIFM TYWHLLNAFT VTVPKDLYVV EYGSNMTIEC KFPVEKQLDL
AALIVYWEME DKNIIQFVHG EEDLKVQHSS YRQRARLLKD QLSLGNAALQ
ITDVKLQDAG VYRCMISYGG ADYKRITVKV NAPYNKINQR ILVVDPVTSE
HELTCQAEGY PKAEVIWTSS DHQVLSGKTT TTNSKREEKL FNVTSTLRIN
TTTNEIFYCT FRRLDPEENH TAELVIPELP LAHPPNERTH LVILGAILLC
LGVALTFIFR LRKGRMMDVK KCGIQDTNSK KQSDTHLEET (SEQ ID NO: 3).

PD-L1 is abundant in a variety of human cancers (Dong et al. (2002) Nat. Med. 8:787-9). The interaction between PD-1 and PD-L1 results in a decrease in tumor infiltrating lymphocytes, a decrease in T-cell receptor mediated proliferation, and immune evasion by the cancerous cells (Dong et al. (2003) J. Mol. Med. 81:281- 7; Blank et al. (2005) Cancer Immunol. Immunother. 54:307-314; Konishi et al. (2004) Clin. Cancer Res. 10:5094-100). Immune suppression can be reversed by inhibiting the local interaction of PD-1 with PD-L1, and the effect is additive when the interaction of PD-1 with PD-L2 is blocked as well.

A PD-1 neutralizing agent is an agent that neutralizes PD-1 or reduces the inhibitory activity of human PD-1. In the context of the present invention, "reduces the inhibitory activity of human PD-1", "neutralizes PD-1" or "neutralizes the inhibitory activity of human PD-1" refers to a process in which PD-1 is inhibited in its signal transduction capacity resulting from the interaction of PD-1 with one or more of its binding partners, such as PD-L1 or PD-L2. An agent that neutralizes the inhibitory activity of PD-1 decreases, blocks, inhibits, abrogates or interferes with signal transduction resulting from the interaction of PD-1 with one or more of its binding partners, such as PD-L1, PD-L2. Such an agent can thereby reduce the negative co-stimulatory signal mediated by or through cell surface proteins expressed on T lymphocytes, so as to enhance T-cell effector functions such as proliferation, cytokine production and/or cytotoxicity. A PD-1 neutralizing agent can interact with PD-1 and/or with one or more of its binding partners, e.g. PD-L1 and PD-L2.

In some embodiments, the PD-1 neutralizing agent is an anti-PD-L1 monoclonal antibody that inhibits the binding of PD-L1 to PD-1. In some embodiments, the PD-1 neutralizing agent is an anti-PD-1 monoclonal antibody that inhibits the binding of PD-1 to PD-L1. In some embodiments, the PD-1 neutralizing agent is an immunoadhesin (e.g., an immunoadhesin comprising an extracellular or PD-1 binding portion of PD-L1 or PD-L2 fused to a constant region (e.g., an Fc region of an immunoglobulin sequence).

In some embodiments, the PD-1 neutralizing agent is an anti-PD-L1 antibody. In some embodiments, the PD-1 neutralizing agent is selected from the group consisting of antibodies YW243.55.S70, MPDL3280A (atezolizumab, Tecentriq^{®}), MDX-1105, and durvalumab (MEDI4736, Imfinzi^{®}). MDX-1105, also known as BMS-936559, is an anti-PD-L1 antibody described in WO2007/005874. Antibody YW243.55.S70 is an anti-PD-L1 described in WO 2010/077634. Examples of anti-PD-L1 antibodies useful for the methods of this invention, and methods for making thereof are also described in WO 2010/077634 A1 and US Patent No. 8,217,149.

In some embodiments, the PD-1 neutralizing agent is a PD-L1 antibody that is durvalumab. Durvalumab (MEDI4736, Imfinzi^{™}) is a human monoclonal antibody directed against human PD-L1 that is capable of blocking the binding of PD-L1 to both the PD-1 and CD80 receptors. Disclosure related to durvalumab can be found in U.S. Pat. Nos. 8,779,108 and 9,493,565. Durvalumab has the heavy and light chains of amino acid sequences SEQ ID NO: 26 and SEQ ID NO: 27, respectively. The heavy chain variable region of durvalumab is shown in SEQ ID NO: 24 and the light chain variable region of durvalumab is shown in SEQ ID NO: 25.

In another embodiment, the PD-1 neutralizing agent is an anti-PD-L1 antibody (or an antigen-binding portion thereof) competing with durvalumab for binding to PD-L1. In some embodiments, the anti-PD-L1 antibody binds to the same epitope as durvalumab. In certain embodiments, the anti-PD-L1 antibody has the same heavy and light chain CDRs as durvalumab.

In one aspect, the PD-1 neutralizing agent (e.g. an agent derived from durvalumab) comprises (i) the heavy chain variable region of SEQ ID NO: 24, or an amino acid sequence at least 50%, 60%, 70%, 80%, 90%, 95%, 98% or 99% identical thereto, and (ii) the light chain variable region of SEQ ID NO: 25, or an amino acid sequence at least 50%, 60%, 70%, 80%, 90%, 95%, 98% or 99% identical thereto. In one aspect, the PD-1 neutralizing agent (e.g. an agent derived from durvalumab) comprises (i) the heavy chain of SEQ ID NO: 26, or an amino acid sequence at least 50%, 60%, 70%, 80%, 90%, 95%, 98% or 99% identical thereto, and (ii) the light chain of SEQ ID NO: 27, or an amino acid sequence at least 50%, 60%, 70%, 80%, 90%, 95%, 98% or 99% identical thereto. In one aspect, the PD-1 neutralizing agent comprises H-CDR1, H-CDR2 and/or H-CDR3 sequences derived from the heavy chain variable region comprising the amino acid sequence of SEQ ID NO: 24. In one aspect, the PD-1 neutralizing agent comprises L-CDR1, L-CDR2 and/or L-CDR3 sequences derived from the light chain variable region comprising the amino acid sequence of SEQ ID NO: 25. Optionally, CDRs are determined according to Kabat.

In one aspect, the PD-1 neutralizing agent comprises the heavy chain H-CDR1, H-CDR2 and H-CDR3 domains having the amino acid sequences of SEQ ID NOS: 28-30, respectively, and the light chain L-CDR1, L-CDR2, L-CDR3 domains having the amino acid sequences of SEQ ID NOS: 31-33, respectively.
Heavy chain variable region of Durvalumab: 
EVQLVESGGGLVQPGGSLRLSCAASGFTFSRYWMSWVRQAPGKGLEWVANIKQDGSEKYYVDSV
KGRFTISRDNAKNSLYLQMNSLRAEDTAVYYCAREGGWFGELAFDYWGQGTLVTVSS
(SEQ ID NO: 24)
Light chain variable region of Durvalumab 
EIVLTQSPGTLSLSPGERATLSCRASQRVSSSYLAWYQQKPGQAPRLLIYDASSRATGIPDRFSGSGS
GTDFTLTISRLEPEDFAVYYCQQYGSLPWTFGQGTKVEIK (SEQ ID NO: 25)
Heavy chain of Durvalumab (variable region underlined) 
EVQLVESGGGLVQPGGSLRLSCAASGFTFSRYWMSWVRQAPGKGLEWVANIKQDGSEKYYVDSV
KGRFTISRDNAKNSLYLQMNSLRAEDTAVYYCAREGGWFGELAFDYWGQGTLVTVSSAS
TKGPSVFPLAPSSKSTSGGTAALGCLVKDYFPEPVTVSWNSGALTSGVHTFPAVLQSSGLYSLSSVV
TVPSSSLGTQTYICNVNHKPSNTKVDKRVEPKSCDKTHTCPPCPAPEFEGGPSVFLFPPKPKDTLMI
SRTPEVTCVVVDVSHEDPEVKFNWYVDGVEVHNAKTKPREEQYNSTYRVVSVLTVLHQDWLNGKEY
KCKVSNKALPASIEKTISKAKGQPREPQVYTLPPSREEMTKNQVSLTCLVKGFYPSDIAVEWESNGQ
PENNYKTTPPVLDSDGSFFLYSKLTVDKSRWQQGNVFSCSVMHEALHNHYTQKSLSLSPGK
(SEQ ID NO: 26)
Light chain of Durvalumab (variable region underlined)

EIVLTQSPGTLSLSPGERATLSCRASQRVSSSYLAWYQQKPGQAPRLLIYDASSRATGIPDRFSGSGS
GTDFTLTISRLEPEDFAVYYCQQYGSLPWTFGQGTKVEIKRTVAAPSVFIFPPSDEQLKSGTASVVCL
LNNFYPREAKVQWKVDNALQSGNSQESVTEQDSKDSTYSLSSTLTLSKADYEKHKVYACEVTHQGL
SSPVTKSFNRGEC (SEQ ID NO: 27)

Durvalumab, Heavy chain CDRs:
H-CDR1: GFTFSRYWMS (SEQ ID NO: 28)
H-CDR2: NIKQDGSEKYYVDSVKG (SEQ ID NO: 29)
H-CDR3: EGGWFGELAFDY SEQ ID NO: 30)

Durvalumab, Light chain CDRs:
L-CDR1: RASQRVSSSYLA (SEQ ID NO: 31)
L-CDR2: DASSRAT (SEQ ID NO: 32)
L-CDR3: QQYGSLPWT (SEQ ID NO: 33)

In another embodiment, the PD-1 neutralizing agent is an anti-PD-L1 antibody that is atezolizumab (MPDL3280A, Tecentriq^{®}, CAS Registry Number: 1422185-06-5). Optionally, the anti-PD-L1 antibody comprises a heavy chain variable region comprising the amino acid sequence: 


(SEQ ID NO: 34)
 or 
EVQLVESGGGLVQPGGSLRLSCAASGFTFSDSWIHWVRQAPGKGLEWVAWISPYGGSTYYADSVKG
RFTISADTSKNTAYLQMNSLRAEDTAVYYCARRHWPGGFDYWGQGTLVTVSSASTK
(SEQ ID NO: 35)
 and a light chain variable region comprising the amino acid sequence:

DIQMTQSPSSLSASVGDRVTITCRASQDVSTAVAWYQQKPGKAPKLLIYSASFLYSGVPSRFS
GSGSGTDFTLTISSLQPEDFATYYCQQYLYHPATFGQGTKVEIKR
(SEQ ID NO: 36).

In one aspect, the PD-1 neutralizing agent comprises (i) a heavy chain or heavy chain variable region of SEQ ID NO: 37, or an amino acid sequence at least 50%, 60%, 70%, 80%, 90%, 95%, 98% or 99% identical thereto, and (ii) a light chain or light chain variable region of SEQ ID NO: 38, or an amino acid sequence at least 50%, 60%, 70%, 80%, 90%, 95%, 98% or 99% identical thereto.

EVQLVESGGGLVQPGGSLRLSCAASGFTFSDSWIHWVRQAPGKGLEWVAWISPYGGSTYYADSVKGR
FTISADTSKNTAYLQMNSLRAEDTAVYYCARRHWPGGFDYWGQGTLVTVSSASTKGPSVFPLA
PSSKSTSGGTAALGCLVKDYFPEPVTVSWNSGALTSGVHTFPAVLQSSGLYSLSSVVTVPSSSL
GTQTYICNVNHKPSNTKVDKKVEPKSCDKTHTCPPCPAPELLGGPSVFLFPPKPKDTLMISRTPEV
TCVVVDVSHEDPEVKFNWYVDGVEVHNAKTKPREEQYASTYRVVSVLTVLHQDWLNGKEYKCK
VSNKALPAPIEKTISKAKGQPREPQVYTLPPSREEMTKQVSLTCLVKGFYPSDIAVEWESNGQPEN
YKTTPPVLDSDGSFFLYSKLTVDKSRWQQGNVFSCSVMHEALHNHYTQKSLSLSPG
(SEQ ID NO: 37)

DIQMTQSPSSLSASVGDRVTITCRASQDVSTAVAWYQQKPGKAPKLLIYSASFLYSGVPSRFSGS
GSGTDFTLTISSLQPEDFATYYCQQYLYHPATFGQGTKVEIKRTVAAPSVFIFPPSDEQLKSGTASVV
CLLNFYPREAKVQWKVDNALQSGNSQESVTEQDSKDSTYSLSSTLTLSKADYEKH
KVYACEVTHQGLSSPVTKSFNRGEC (SEQ ID NO: 38).

In some embodiments, the PD-1 neutralizing agent is an anti-PD-1 antibody that inhibits the binding of PD-1 to PD-L1. In one embodiment, the anti-PD-1 antibody is nivolumab. Nivolumab (also known as "OPDIVO^{®}"; formerly designated 5C4, BMS-936558, MDX-1106, or ONO-4538) is a fully human IgG4 (S228P) PD-1 immune checkpoint inhibitor antibody that selectively prevents interaction with PD-1 ligands (PD-L1 and PD-L2), thereby blocking the down-regulation of antitumor T-cell functions (U.S. Pat. No. 8,008,449; Wang et al., 2014 Cancer Immunol Res. 2(9):846-56). In another embodiment, the anti-PD-1 antibody or fragment thereof competes with nivolumab for binding to PD-1. In some embodiments, the anti-PD-1 antibody binds to the same epitope as nivolumab. In certain embodiments, the anti-PD-1 antibody has the same heavy and light chain CDRs as nivolumab.

In another embodiment, the anti-PD-1 antibody is pembrolizumab. Pembrolizumab (also known as "KEYTRUIDA^{®}", lambrolizumab, and MK-3475) is a humanized monoclonal IgG4 antibody directed against human cell surface receptor PD-1. Pembrolizumab is described, for example, in U.S. Pat. No. 8,900,587). Pembrolizumab has been approved by the FDA for the treatment of relapsed or refractory melanoma and advanced NSCLC. In another embodiment, the anti-PD-1 antibody (or an antigen-binding portion thereof) competes with pembrolizumab for binding to PD-1. In some embodiments, the anti-PD-1 antibody binds to the same epitope as pembrolizumab. In certain embodiments, the anti-PD-1 antibody has the same heavy and light chain CDRs as pembrolizumab.

A NKG2A neutralizing agent or aPD-1 neutralizing agent such as an antibody can be incorporated in a pharmaceutical formulation in a concentration from 1 mg/ml to 500 mg/ml, wherein said formulation has a pH from 2.0 to 10.0. The NKG2A neutralizing agent and the PD-1 neutralizing agent can be comprised in the same or separate pharmaceutical formulations. The formulation may further comprise a buffer system, preservative(s), tonicity agent(s), chelating agent(s), stabilizers and surfactants. In one embodiment, the pharmaceutical formulation is an aqueous formulation, i.e., formulation comprising water. Such formulation is typically a solution or a suspension. In a further embodiment, the pharmaceutical formulation is an aqueous solution. The term "aqueous formulation" is defined as a formulation comprising at least 50 %w/w water. Likewise, the term "aqueous solution" is defined as a solution comprising at least 50 %w/w water, and the term "aqueous suspension" is defined as a suspension comprising at least 50 %w/w water.

In another embodiment, the pharmaceutical formulation is a freeze-dried formulation, whereto the physician or the patient adds solvents and/or diluents prior to use.

In another embodiment, the pharmaceutical formulation is a dried formulation (e.g. freeze-dried or spray-dried) ready for use without any prior dissolution.

In a further aspect, the pharmaceutical formulation comprises an aqueous solution of such an antibody, and a buffer, wherein the antibody is present in a concentration from 1 mg/ml or above, and wherein said formulation has a pH from about 2.0 to about 10.0.

In another embodiment, the pH of the formulation is in the range selected from the list consisting of from about 2.0 to about 10.0, about 3.0 to about 9.0, about 4.0 to about 8.5, about 5.0 to about 8.0, and about 5.5 to about 7.5.

In a further embodiment, the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative embodiment of the invention.

In a further embodiment, the formulation further comprises a pharmaceutically acceptable preservative. In a further embodiment, the formulation further comprises an isotonic agent. In a further embodiment, the formulation also comprises a chelating agent. In a further embodiment of the invention the formulation further comprises a stabilizer. In a further embodiment, the formulation further comprises a surfactant. For convenience reference is made to Remington: The Science and Practice of Pharmacy, 19th edition, 1995.

It is possible that other ingredients may be present in the pharmaceutical formulation of the present invention. Such additional ingredients may include wetting agents, emulsifiers, antioxidants, bulking agents, tonicity modifiers, chelating agents, metal ions, oleaginous vehicles, proteins (e.g., human serum albumin, gelatine or proteins) and a zwitterion (e.g., an amino acid such as betaine, taurine, arginine, glycine, lysine and histidine). Such additional ingredients, of course, should not adversely affect the overall stability of the pharmaceutical formulation of the present invention.

Administration of pharmaceutical compositions according to the invention may be through several routes of administration, for example, intravenous. Suitable antibody formulations can also be determined by examining experiences with other already developed therapeutic monoclonal antibodies.

Also provided are kits for use in the treatment of a MSS-CRC, which include:
(i) a pharmaceutical composition containing a NKG2A neutralizing agent such as an anti-NKG2A antibody, and a PD-1 neutralizing agent such as an anti-PD-1 antibody or an anti-PD-L1 antibody, or
(ii) a first pharmaceutical composition containing a PD-1 neutralizing agent such as an anti-PD-1 antibody or an anti-PD-L1 antibody, and a second pharmaceutical composition containing a NKG2A neutralizing agent such as an anti-NKG2A antibody, or
(iii) a pharmaceutical composition containing a NKG2A neutralizing agent such as an anti-NKG2A antibody, wherein the treatment comprises administering said NKG2A neutralizing agent with a PD-1 neutralizing agent such as an anti-PD-1 antibody or an anti-PD-L1 antibody, or
(iv) a pharmaceutical composition containing a PD-1 neutralizing agent such as an anti-PD-1 antibody or an anti-PD-L1 antibody, wherein the treatment comprises administering said PD-1 neutralizing agent with a NKG2A neutralizing agent such as an anti-NKG2A antibody.

A pharmaceutical composition may optionally be specified as comprising a pharmaceutically-acceptable carrier. A NKG2A or PD-1 neutralizing agent may optionally be specified as being present in a therapeutically effective amount adapted for use in any of the methods herein. The kits optionally also can include instructions, e.g., comprising administration schedules, to allow a practitioner (e.g., a physician, nurse, or patient) to administer the composition contained therein to a patient having cancer (e.g., a solid tumor, in particular a tumor that is not DNA mismatch-repair defective and/or that does not have microsatellite instability detected in two or more microsatellite makers). In any embodiment, a kit optionally can include instructions to administer said NKG2A neutralizing agent simultaneously, separately, or sequentially with said PD-1 neutralizing agent. The kit also can include a syringe.

Optionally, the kits include multiple packages of the single-dose pharmaceutical compositions each containing an effective amount of the NKG2A neutralizing agent, and/or the PD-1 neutralizing agent such as an anti-PD-1 or PD-L1 antibody, for a single administration in accordance with the methods provided above. Instruments or devices necessary for administering the pharmaceutical composition(s) also may be included in the kits. For instance, a kit may provide one or more pre-filled syringes containing an amount of the anti-NKG2A, anti-PD-1 or anti-PD-L1 antibody.

An aspect useful for understanding the invention relates to a kit for treating a cancer or a tumor in a human patient, wherein said cancer or tumor is not MSI-H and/or not DNA mismatch repair defective, the kit comprising:
(a) a dose of an anti-NKG2A antibody comprising the H-CDR1, H-CDR2 and H-CDR3 domains of a heavy chain variable region having the sequence set forth in any of SEQ ID NOS: 4-8, and the L-CDR1, L-CDR2 and L-CDR3 domains of a light chain variable region having the sequence set forth in SEQ ID NO: 9; and/or
(b) a dose of an anti-PD-1 antibody or an anti-PD-L1 antibody, optionally a dose of durvalumab, optionally a dose of an anti-PD-L1 antibody comprising the heavy and light chain H-CDR1, H-CDR2 and H-CDR3 domains of durvalumab, optionally the H-CDR1, H-CDR2 and H-CDR3 domains of a heavy chain variable region having the sequence set forth in SEQ ID NO: 24, and the L-CDR1, L-CDR2 and L-CDR3 domains of a light chain variable region having the sequence set forth in SEQ ID NO: 25; and
(c) optionally, instructions for using said anti-NKG2A antibody and/or said anti-PD-1 or PD-L1 antibody in any of the methods described herein.

An aspect useful for understanding the invention relates to a kit for treating a cancer or a tumor in a human patient, wherein said cancer or tumor is not MSI-H and/or not DNA mismatch repair defective, the kit comprising:
(a) a dose of an anti-NKG2A antibody comprising the heavy chain H-CDR1, H-CDR2 and H-CDR3 domains having the sequences of SEQ ID NOS: 16-18, and the light chain L-CDR1, L-CDR2 and L-CDR3 domains having the sequences of SEQ ID NOS: 19-21, respectively; and/or
(b) a dose of an anti-PD-L1 antibody comprising the heavy chain H-CDR1, H-CDR2 and H-CDR3 domains having the amino acid sequences of SEQ ID NOS: 28-30, and the light chain L-CDR1, L-CDR2 and L-CDR3 domains having the amino acid sequences of SEQ ID NOS: 31-33, respectively; and
(c) optionally, instructions for using said anti-NKG2A antibody and/or said anti-PD-1 or PD-L1 antibody in any of the methods described herein.

### Diagnostics, prognostics, and treatment of malignancies

Described are methods useful in the diagnosis, prognosis, monitoring and treatment of a cancer, particularly colorectal cancer, optionally advanced recurrent or metastatic colorectal cancer, characterized by tumors that are not DNA mismatch repair defective and/or that are microsatellite stable. Colorectal cancer (CRC) as used herein refers to colon cancer, rectal cancer, and colorectal cancer (cancer of both the colon and rectal areas).

Microsatellites are repeated sequences of DNA distributed throughout the genome. Although the length of these microsatellites is highly variable from person to person, each individual has microsatellites of a set length. These repeated sequences are common, and normal. The most common microsatellite in humans is a dinucleotide repeat of CA, which occurs tens of thousands of times across the genome. In cells with mutations in DNA repair genes, however, some of these sequences accumulate errors and become longer or shorter. The appearance of abnormally long or short microsatellites in an individual's DNA is referred to as microsatellite instability (MSI). Microsatellite instability is the condition of genetic hypermutability that results from impaired DNA mismatch repair (MMR). The presence of microsatellite instability (MSI) represents phenotypic evidence that MMR is not functioning normally. The absence of microsatellite instability is termed microsatellite stability (MSS).

MSI is a key factor in several cancers including colorectal, endometrial, ovarian and gastric cancers (Soreide et al. (2006) The British Journal of Surgery 93:395-406; Ali-Fehmi et al. (2006) International Journal of Gynecological Pathology 25:223-229; Vauhkonen et al. (2006) Clinical Gastroenterology 20:651-674).

Colorectal cancer studies have demonstrated two mechanisms for MSI occurrence. The first is in hereditary nonpolyposis colorectal cancer (HNPCC) or Lynch Syndrome, where an inherited mutation in a DNA mismatch-repair gene causes a microsatellite repeat replication error to go unfixed. The replication error results in a frameshift mutation that inactivates or alters major tumor suppressor genes and, ultimately, the prevention of cancer. The second mechanism whereby MSI causes colorectal cancer is an epigenetic change which silences an essential DNA mismatch-repair gene. In both cases, microsatellite insertions and deletions within tumor suppressor gene coding regions result in uncontrolled cell division and tumor growth.

Five markers have been recommended by the National Cancer Institute to screen for MSI in HNPCC tumors (often called "Bethesda markers"). These five markers of MSI presence are: two mononucleotide repeats BAT25 and BAT26, and three dinucleotide repeats D5S346, D2S123, and D17S250 (Umar et al (2004) Journal of the National Cancer Institute 96:261-268). Generally, MSI detection in two of the five "Bethesda markers" is considered a positive result or high probability of MSI (MSI-High or MSI-H). Standard methods for detecting MSI in biological samples include the use of Promega^{™'}s microsatellite instability assay (MSI Analysis System) that includes five mononucleotide markers chosen for their sensitivity and specificity, these five markers are: BAT-25, BAT-26, NR-21, NR-24 and MONO27 (Bacher et al. (2004) Disease Markers 20:237-250).

In most cases, the genetic basis for instability in MSI tumors is an inherited germline alteration in any one or more of the five human MMR genes: MSH2, MLH1, MSH6, PMS2, and PMS1.

Another MSI, called elevated microsatellite alterations at selected tetranucleotide repeats (EMAST), was recently discovered. However, EMAST is unique in that it is not derived from MMR, and it is commonly associated with TP53 mutations (Boland et al. (2010) Gastroenterology 138 (6): 2073-2087).

Thus, microsatellite instability in a tumor can be determined by assessing microsatellite markers and/or MMR genes.

In certain embodiments, the individual who is treated with the combination of NKG2A-neutralizing agent and PD-1 neutralizing agent has no instability (MSS) and has no alteration (e.g. mutation, deficiency in expression) in any of the MSH2, MLH1, MSH6 and PMS2 gene or protein (optionally further PMS1).

In some embodiments, the invention includes a method of treating a tumor in an individual, e.g., colorectal tumor, comprising (i) identifying an individual who has a tumor that is not DNA mismatch repair defective, and (ii) administering to the individual an effective amount of a NKG2A-neutralizing agent and an effective amount of a PD-1 neutralizing agent. Optionally, the individual has a tumor that has no microsatellite instability (is MSS stable) and has no alternation in any of MSH2, MLH1, MSH6 and PMS2 genes or proteins.

The DNA mismatch repair status of a tumor, optionally the MMR status and/or microsatellite status in an individual can be measured prior to administering any composition or utilizing any method disclosed herein.

A biological sample from an individual, for example from a biopsy, can be obtained and assessed. MMR status and/or microsatellite status can be determined by any methods known in the art, see, e.g., Umar et al. Journal of the National Cancer Institute 2004;96(4):261-268 and Bacher et al. Disease Markers 2004; 20:237-250. In one embodiment, MMR status is assessed by immunohistochemical analysis demonstrating the presence or absence of expression of any one or more of the following proteins: MLH1, MSH2, MSH6, or PMS2. In one embodiment, microsatellite status is assessed by detecting high-frequency microsatellite instability in microsatellite markers, for example BAT-25, BAT-26, NR-21, NR-24, MONO27, D5S346, D2S123, and D17S250. In one embodiment, microsatellite instability detected for two or more microsatellite markers, for example for BAT-25, BAT-26, NR-21, NR-24, and/or MONO27, indicates a MSI-H status, while microsatellite instability for a single MSI marker or no instability for any of the MSI markers tested is interpreted as microsatellite instablility-Low (MSI-L) and microsatellite stable (MSS), respectively.

In one embodiment, a tumor that is not DNA mismatch repair defective or that is MSS has no microsatellite instability or microsatellite instability detected at less than two or more microsatellite markers, for example BAT-25, BAT-26, NR-21, NR-24, or MONO27, and no absence of protein expression at any one or more of proteins MLH1, MSH2, MSH6, or PMS2.

In some embodiments, the invention includes a method of treating a tumor in an individual, e.g., colorectal tumor, comprising (i) identifying an individual who has a MSS tumor and (ii) administering to the individual an effective amount of a NKG2A-neutralizing agent, further administering to the individual an effective amount of a PD-1 neutralizing agent. In some embodiments, the invention provides a method of treating a tumor, e.g., a colorectal tumor, comprising (i) identifying an individual who has a tumor that is not a MSI-High (MSI-H) tumor (e.g. a MSS or a MSI-Low tumor) and (ii) administering to the individual an effective amount of a NKG2A-neutralizing agent, further administering to the individual an effective amount of a PD-1 neutralizing agent.

In one embodiment, MSI-H tumors have greater than at least about 30% of unstable MSI markers. In one embodiment, MSI-L tumors do have unstable MSI markers but less than about 10%, less than about 20%, or less than about 30% of the MSI markers of said tumors are unstable MSI markers. In one embodiment, MSS tumors have no unstable MSI marker. In some embodiments, a colorectal cancer is MSI-L when less than about 30%, less than about 20% or less than about 10% of the tested MSI markers exhibit instability. In some embodiments, a colorectal cancer is MSS when none of the tested MSI markers exhibit instability.

In certain embodiments, the present invention is directed to a method of treating a cancer that is MSI-L or MSS.

In certain embodiments, the present invention is directed to a method of treating a cancer comprising 1) identifying the microsatellite status of a tumor and 2) administering a therapy (e.g. a NKG2A-neutralizing agent and a PD-1-neutralizing agent) to the subject based on the microsatellite status. In other embodiments, the subject has MSI-L. In embodiments, the patient is MSI stable.

When treating an individual having a non-DNA mismatch repair defective tumor, an antibody that neutralizes the inhibitory activity of a human NKG2A polypeptide can advantageously be administered according to a treatment regimen described herein, optionally to an individual having a cancer who has received, or who is undergoing, surgery to remove cancer cells. A neutralizing anti-NKG2A antibody, in combination with a PD-1-neutralizing agent, e.g. a neutralizing anti-PD-1 or anti-PD-L1 antibody, to treat subjects afflicted with cancer, particularly CRC and mCRC. In one embodiment, the invention provides an anti-NKG2A antibody, and further an anti-PD-1 antibody in combination, to treat subjects having a solid tumor (e.g., a solid tumor, an advanced refractory solid tumor). In a particular embodiment, the anti-NKG2A antibody comprises a heavy chain variable region of any of SEQ ID NOS: 4-8 and a light chain variable region of SEQ ID NO: 9. In one embodiment, the antibody that neutralizes the inhibitory activity of PD-1 is selected from the group consisting of pembrolizumab, nivolumab, durvalumab and MPDL3280A, in particular durvalumab.

As used herein, adjunctive or combined administration (co-administration) includes simultaneous administration of the compounds in the same or different dosage form, or separate administration of the compounds (e.g., sequential administration). A NKG2A-neutralizing agent is used in combination with a PD-1-neutralizing agent. For example, an anti-NKG2A antibody and an anti-PD-1 or anti-PD-L1 antibody can be simultaneously administered in a single formulation. Alternatively, the NKG2A-neutralizing agent and the PD-1-neutralizing agent can be formulated for separate administration and are administered concurrently or sequentially.

Optionally, an individual may have a cancer that is resistant, has not responded, has relapsed and/or progressed despite (e.g. during or following) surgery and/or treatment with a therapeutic agent, e.g. a chemotherapeutic agent or radiotherapy. In any embodiment herein, treatment response can be defined and/or assessed according to well-known criteria, e.g. Response Evaluation Criteria In Solid Tumors (RECIST), such as version 1.1, see Eisenhauer et al. (2009) Eur. J. Cancer 45:228-247, or Immune-Related Response Criteria (irRC), see Wolchock et al. (2009) Clinical Cancer Research 15:7412-7420.

In another embodiment, the disclosure provides a method for the treatment or prevention of a CRC in an individual having a tumor that is not DNA mismatch repair defective, the method comprising:
a) identifying an individual who has a tumor that is not DNA mismatch-repair defective, optionally obtaining a biological sample comprising tumor cells from the individual and determining whether the tumor is DNA mismatch-repair defective,
b) detecting cells (e.g. tumor cells, tumor infiltrating immune cells, tumor infiltrating macrophages) that express PD-L1 in a sample from the individual, and
c) upon a determination that cells which express PD-L1 are comprised in the sample, administering to the individual an agent that neutralizes the inhibitory activity of NKG2A in combination with an agent that neutralizes the inhibitory activity of PD-1. The PD-L1 reference level can be characterized by any suitable conventionally used reference level. For example, if 1% or more, optionally 5% or more, optionally 10% or more, optionally 50% or more of tumor cells or cells from a tumor tissue sample express PD-L1 (e.g. using an immunohistochemistry-based assay). Example of such assays include the PD-L1 IHC 22C3 assay from pharmDx from Dako Denmark A/S. In this assay, PD-L1 expression level is measured using the tumor proportion score (TPS), the percentage of tumor cells staining for PD-L1 (0% to 100%). Optionally, a reference level is a level for a non-high PD-L1 expression, optionally wherein less 50% tumor cells express PD-L1 (e.g., the patients have a TPS of less than 50%).

The treatment regimens and methods described herein may be used with or without a prior step of detecting the expression HLA-E on cells in a biological sample obtained from an individual (e.g. a biological sample comprising cancer cells, cancer tissue or canceradjacent tissue). In one embodiment, the cancer treated with the methods disclosed herein is a cancer characterized by high levels of HLA-E. However, it will be appreciated that a patient having a cancer can be treated with the NKG2A neutralizing agent with or without a prior detection step to assess expression of HLA-E on the surface of tumor cells. Advantageously, the treatment methods can comprises a step of detecting a HLA-E nucleic acid or polypeptide in a biological sample of a tumor (e.g. on a tumor cell) from an individual. A determination that a biological sample expresses HLA-E (e.g. prominently expresses; expresses HLA-E at a high level, high intensity of staining with an anti-HLA-E antibody, compared to a reference) indicates that the individual has a cancer that may have a strong benefit from treatment with an agent that inhibits NKG2A. In one embodiment, the method comprises determining the level of expression of a HLA-E nucleic acid or polypeptide in a biological sample and comparing the level to a reference level (e.g. a value, weak cell surface staining, etc.) corresponding to a healthy individual. A determination that a biological sample expresses an HLA-E nucleic acid or polypeptide at a level that is increased compared to the reference level may indicate that the individual has a cancer that can be treated with an agent that inhibits NKG2A.

Determining whether an individual has cancer cells that express an HLA-E polypeptide can for example comprise obtaining a biological sample (e.g. by performing a biopsy) from the individual that comprises cancer cells, bringing said cells into contact with an antibody that binds an HLA-E polypeptide, and detecting whether the cells express HLA-E on their surface. Optionally, determining whether an individual has cancer cells that express HLA-E comprises conducting an immunohistochemistry assay. Optionally determining whether an individual has cancer cells that express HLA-E comprises conducting a flow cytometry assay.

In the treatment methods, an NKG2A-neutralizing agent is administered in combination with an anti-PD-1 or anti-PD-L1 antibody. The NKG2A-neutralizing agent and anti-PD-1 or anti-PD-L1 antibody can be administered separately, together or sequentially, or in a cocktail. In some embodiments, the NKG2A-neutralizing agent is administered prior to the administration of the anti-PD-1 or anti-PD-L1 antibodies. For example, the NKG2A-neutralizing agent can be administered approximately 0 to 30 days prior to the administration of the anti-PD-1 or anti-PD-L1 antibodies. In some embodiments, antibody NKG2A-neutralizing agent is administered from about 30 minutes to about 2 weeks, from about 30 minutes to about 1 week, from about 1 hour to about 2 hours, from about 2 hours to about 4 hours, from about 4 hours to about 6 hours, from about 6 hours to about 8 hours, from about 8 hours to 1 day, or from about 1 to 5 days prior to the administration of the anti-PD-1 or anti-PD-L1 antibodies. In some embodiments, a NKG2A-neutralizing agent is administered concurrently with the administration of the anti-PD-1 or anti-PD-L1 antibodies. In some embodiments, a NKG2A-neutralizing agent is administered after the administration of the anti-PD-1 or anti-PD-L1 antibodies. For example, a NKG2A-neutralizing agent can be administered approximately 0 to 30 days after the administration of the anti-PD-1 or anti-PD-L1 antibodies. In some embodiments, a NKG2A-neutralizing agent is administered from about 30 minutes to about 2 weeks, from about 30 minutes to about 1 week, from about 1 hour to about 2 hours, from about 2 hours to about 4 hours, from about 4 hours to about 6 hours, from about 6 hours to about 8 hours, from about 8 hours to 1 day, or from about 1 to 5 days after the administration of the anti-PD-1 or anti-PD-L1 antibodies.

Exemplary treatment protocols for treating a human with an anti-NKG2A antibody include, for example, administering to the patient an effective amount of an antibody that inhibits NKG2A, wherein the method comprises at least one administration cycle in which at least one dose of the anti-NKG2A antibody is administered at a dose of 1-10 mg/kg body weight. In one embodiment, the administration cycle is between 2 weeks and 8 weeks.

Exemplary treatment protocols for treating a human with an anti-NKG2A antibody include, for example, administering to the patient an effective amount of each of an antibody that inhibits NKG2A and an antibody that neutralizes the inhibitory activity of human PD-1, wherein the method comprises at least one administration cycle in which at least one dose of the anti-NKG2A antibody is administered at a dose of 0.1-10 mg/kg body or 1-10 mg/kg body weight and at least one dose of the anti-PD-1 or anti-PD-L1 antibody is administered at a dose of 1-20 mg/kg body weight. In one embodiment, the administration cycle is between 2 weeks and 8 weeks.

In one embodiment, the method comprises at least one administration cycle, wherein the cycle is a period of eight weeks or less, wherein for each of the at least one cycles, two, three or four doses of the anti-NKG2A antibody are administered at a dose of 1-10 mg/kg body weight. In one embodiment, each cycle further comprises the administration of two, three or four doses of the anti-PD-1 or anti-PD-L1 antibody at a dose of 1-20 mg/kg body weight.

The anti-NKG2A antibody can advantageously be administered in an amount that achieves a concentration in circulation that is at least 10, 20, or 30 times higher than the concentration required for substantially full (e.g., 90%, 95%) receptor saturation (e.g., as assessed by titrating anti-NKG2A antibody on NKG2A-expressing cells, for example in PBMC), or optionally in an amount that achieves a concentration in a extravascular tissue (e.g. the tumor tissue or environment) that is at least 10, 20, or 30 times higher than the concentration required for substantially full receptor saturation (e.g., as assessed by titrating anti-NKG2A antibody on NKG2A-expressing cells, for example in PBMC).

NKG2A+ NK cell response can be assessed using a suitable assay of cytotoxic activity of NKG2A-expressing NK cells toward HLA-E expressing target cells. Examples include assays based on markers of NK cell activation, for example CD107 or CD137 expression. Advantageously an amount of anti-NKG2A antibody can be administered so at to achieve and/or maintain a continuous (minimum) tissue concentration of at least 10 µg/ml. For example, the blood concentration to be achieved and/or maintained in order to achieve/maintain 10 µg/ml in a tissue can be between 100-110 µg/ml, 100-120 µg/ml, 100-130 µg/ml, 100-140 µg/ml, 100-150 µg/ml, 100-200 µg/ml, 100-250 µg/ml or 100-300 µg/ml.

Exemplary treatment protocols for an anti-NKG2A antibody such as humZ270 (monalizumab) used in the Examples herein having an EC₁₀₀ for NKG2A+ NK cell response of about 10 µg/ml comprise at least one administration cycle in which at least one dose of the anti-NKG2A antibody is administered at a dose of about 10 mg/kg, optionally 2-10 mg/kg, optionally 4-10 mg/kg, optionally 6-10 mg/kg, optionally 2-6 mg/kg, optionally 2-8 mg/kg, or optionally 2-4 mg/kg body weight. Optionally, at least 2, 3, 4, 5, 6, 7 or 8 doses of the anti-NKG2A antibody are administered. In one embodiment, the administration cycle is between 2 weeks and 8 weeks. In one embodiment, the administration cycle is 8 weeks. In one embodiment, the administration cycle is 8 weeks and comprises administering one dose of the anti-NKG2A antibody every two weeks (i.e. a total of four doses).

In one aspect of any of the embodiments herein, the anti-NKG2A antibody is administered once about every two weeks.

Exemplary treatment protocols for use with an anti-NKG2A antibody include for example, administering to the patient an anti-NKG2A antibody two times per month in an amount effective to maintain a continuous blood concentration of anti-NKG2A antibody of at least 40 µg/ml between at least two successive administrations of the anti-NKG2A antibody is between 2-10 mg/kg, optionally 2-6 mg/kg, optionally 2-4 mg/kg, optionally about 4 mg/kg body weight. These doses can optionally be administered so as to provide for continued blood concentration of anti-NKG2A antibody of at least 40 µg/ml throughout the treatment cycle. Achieving blood concentration of anti-NKG2A antibody of 40 µg/ml is expected to provide a tissue (e.g., extravascular tissue, tumor environment) concentration of about 4 µg/ml, in turn corresponding to the EC₅₀ for an antibody such as humanized Z270 (monalizumab).

Exemplary treatment protocols for use with an anti-NKG2A antibody include for example, administering to the patient an effective amount of an anti-NKG2A antibody, wherein the antibody is administered 2 times per month and the amount effective to maintain a continuous blood concentration of anti-NKG2A antibody of at least 100 µg/ml between at least two successive administrations of the anti-NKG2A antibody is between 4-10 mg/kg, optionally 4-6 mg/kg, optionally 4-8 mg/kg, optionally about 4 mg/kg, optionally about 6 mg/kg, optionally about 8 mg/kg, or optionally about 10 mg/kg. These doses can optionally be administered so as to provide for continued blood concentration of anti-NKG2A antibody of at least 100 µg/ml throughout the treatment cycle. Achieving blood concentration of anti-NKG2A antibody of 100 µg/ml is expected to provide a tissue (e.g., extravascular, tumor environment) concentration of about 10 µg/ml, in turn corresponding to the EC₁₀₀ for an antibody such as humanized Z270.

In certain embodiments, a dose (e.g. each dose) of the anti-NKG2A antibody (e.g. monalizumab) is administered at 0.1, 0.3, 1, 3, 4, 6, 8 or 10 mg/kg. In certain embodiments, a dose (e.g. each dose) of the anti-NKG2A antibody (e.g. monalizumab) is administered at a fixed dose of 7.5 mg, 22.5 mg, 75 mg, 225 mg or 750 mg, optionally administered every two weeks. In certain embodiments, a dose (e.g. each dose) of the anti-PD-1 antibody is administered at 1-20 mg/kg, optionally at 10 mg/kg. In certain embodiments, a dose (e.g. each dose) of the anti-PD-L1 antibody (e.g. durvalumab) is administered at 10, 15, 20 or 25 mg/kg, optionally at 750 mg total dose, optionally at 1500 mg total dose optionally administered every 4 weeks. In certain embodiments, the combined therapy permits the anti-PD-1 or PD-L1 antibody to be administered at a lower dose.

In one embodiment, the anti-NKG2A antibody and anti-PD-1 or anti-PD-L1 antibody are administered at the following doses:
(a) 0.1-10 mg/kg anti-NKG2A antibody and (i) 1-10 mg/kg of anti-PD-1 antibody or (ii) 1-20 mg/kg of anti-PD-L1 antibody;
(b) 1-10 mg/kg anti-NKG2A antibody and (i) 1-10 mg/kg of anti-PD-1 antibody or (ii) 1-20 mg/kg of anti-PD-L1 antibody;
(c) 225 mg anti-NKG2A antibody (e.g. monalizumab) and 750 mg/kg of anti-PD-L1 antibody (e.g. durvalumab);
(d) 750 mg anti-NKG2A antibody (e.g. monalizumab) and 750 mg/kg of anti-PD-L1 antibody (e.g. durvalumab);
(e) 225 mg anti-NKG2A antibody (e.g. monalizumab) and 1500 mg/kg of anti-PD-L1 antibody (e.g. durvalumab); or
(f) 750 mg anti-NKG2A antibody (e.g. monalizumab) and 1500 mg/kg of anti-PD-L1 antibody (e.g. durvalumab).

In one aspect of any of the embodiments herein, the anti-NKG2A antibody is administered once about every two weeks, optionally once every four weeks. In one aspect of any of the embodiments herein, the anti-PD-1 or anti-PD-L1 antibody is administered once about every four weeks.

In one embodiment the anti-PD-1 or anti-PD-L1 antibody and/or the anti-NKG2A antibody are administered by i.v. In one embodiment the anti-PD-1 or anti-PD-L1 antibody is administered every four weeks and the anti-NKG2A antibody is administered every two weeks, wherein every four weeks the anti-PD-1 or anti-PD-L1 antibody and NKG2A antibody are administered on the same day, optionally further by i.v.

An aspect useful for understanding the invention relates to a method for assessing whether an individual is suitable for treatment with an agent that inhibits NKG2A (and optionally further an agent that neutralizes the inhibitory activity of human PD-1), the method comprising assessing tumor DNA mismatch repair status in a biological sample from an individual. A determination that the individual has a tumor that is not DNA mismatch repair defective indicates that the patient has a cancer that can be treated with an agent that inhibits NKG2A, optionally further in combination with an agent that neutralizes the inhibitory activity of human PD-1. In one embodiment, the method is used to assess whether an individual is suitable for treatment with an agent that inhibits NKG2A and an agent that neutralizes the inhibitory activity of human PD-1 administered according to the administration regimens disclosed herein.

In another aspect, provided is a method of reducing the risk of cancer progression, reducing the risk of further cancer in CRC, optionally mCRC, and/or providing a therapeutic regimen for reducing cancer progression in a human individual, which comprises administering to the patient an amount of a NKG2A-neutralizing agent and a PD-1 neutralizing agent in a dosage and frequency according to the disclosure. In a further aspect, provided is a method of promoting remission of a cancer in an individual, such as a human patient, in particular a colorectal cancer, comprising administering a pharmaceutical composition comprising a NKG2A-neutralizing agent and a PD-1 neutralizing agent, to the individual, in a dosage and frequency according to the disclosure, so as to promote cancer remission in the individual. In a further aspect, provided is a method of preventing recurrence of a cancer, in particular colorectal cancer, in an individual, such as a human patient, whose cancer is in remission following a preceding anti-cancer treatment, comprising administering to the individual a composition comprising a NKG2A-neutralizing agent and a PD-1 neutralizing agent, in a dosage and frequency according to the disclosure, so as to promote cancer remission in the individual. In a further aspect, provided is a method of increasing the likelihood of survival over a relevant period in a human patient diagnosed with cancer, in particular a colorectal cancer, comprising administering to the patient a pharmaceutical composition comprising a NKG2A-neutralizing agent and a PD-1-neutralizing agent. In another aspect, provided is a method for improving the quality of life of a cancer patient comprising administering to the patient a pharmaceutical composition comprising a NKG2A-neutralizing agent and a PD-1 neutralizing agent in an amount effective to improve the quality of life thereof. In a further aspect, methods described herein can be applied to significantly reduce the number of cancer cells in a human, such that, for example, the total number of cancer cells is reduced. In a related sense, provided is a method for killing (e.g. either directly or indirectly causing death of) cancer cells in a mammal, such as a human cancer patient, in particular colorectal cancer cells. In still other embodiments of the methods described herewith, said individual has a tumor that is not MSI-High (MSI-H) and/or not DNA mismatch repair (MMR) defective.

The NKG2A-neutralizing agent in combination with the PD-1 neutralizing agent can be administered in combined administration (co-administration) with one or more additional therapeutic agents or therapies.

### EXAMPLES

### Example 1: Dose finding is a Phase 1 multicenter, open-label, single-arm dose-escalation and dose-expansion study of durvalumab in combination with monalizumab

A Phase 1, multicenter, open-label, single-arm dose-escalation and dose-expansion study of durvalumab in combination with monalizumab (see WHO Drug Information Vol. 30, No. 1, 2016; also referred to as IPH2201; antibody having the heavy chain of SEQ ID NO: 11 and a light chain of SEQ ID NO: 15) was conducted to evaluate the safety, tolerability, PK, immunogenicity, pharmacodynamics, and antitumor activity in adult subjects with selected advanced solid tumors. The study consisted of 2 parts: a dose escalation and a dose expansion.

Subjects received durvalumab and monalizumab via 2 separate IV infusions. Subjects received durvalumab and monalizumab until unacceptable toxicity, documentation of confirmed progressive disease (PD), or documentation of subject withdrawal for another reason.

Inclusion Criteria included:
1. Subjects must have histologic documentation of advanced recurrent or metastatic cancer.
2. Subjects must have received and have progressed or are refractory to at least one line of standard systemic therapy in the recurrent/metastatic setting, with selected advanced solid tumors.
3. Subjects must have at least one lesion that is measurable by RECIST v1.1

Exclusion Criteria were as follows:"
1. Prior treatment with immunotherapy agents. Prior treatment with antitumor vaccines may be permitted upon discussion with the medical monitor.
2. Prior participation in clinical studies that include durvalumab alone or in combination, where the study has registrational intent and the analyses for the primary endpoint have not yet been completed
3. Receipt of any conventional or investigational anticancer therapy within 4 weeks prior to the first dose of durvalumab and monalizumab
4. Any concurrent chemotherapy, immunotherapy, biologic or hormonal therapy for cancer treatment. Concurrent use of hormones for non-cancer-related conditions is acceptable. Local treatment of isolated lesions for palliative intent is acceptable beyond the DLT-evaluation period with prior consultation and in agreement with the medical monitor.
5. Current or prior use of immunosuppressive medication within 14 days before the first dose.

Subjects in sequential cohorts received durvalumab (1500 mg every 4 weeks (Q4W)) in combination with monalizumab at 1 of 4 planned dose levels (22.5, 75, 225, or 750 mg every 2 weeks (Q2W).

In the 15 patients treated with monalizumab plus durvalumab in escalation, there were no treatment-related adverse events (TRAEs) that led to discontinuations, no grade 3/4 TRAEs, no DLTs, and no deaths; MTD was not reached. Any grade TRAEs were observed in 12 patients (80%); most frequent was diarrhea (n=4). Safety in the 40 patients in expansion was similar to escalation: 19 patients (48%) had any grade TRAEs, 1 patient had a grade 3/4 TRAE (sepsis). PK of both drugs in combination showed no interactions. Monalizumab PK approached linearity at the highest dose level, which was thus chosen for expansion.

At the end of the dose-escalation part of the trial, the maximum tolerated dose (MTD) was not reached and Cohort 4 dosing of monalizumab (750 mg Q2W) and durvalumab (1500 mg Q4W) was deemed safe by the dose escalation committee (DEC). Those doses are then employed in the dose expansion part of the study which is currently on-going. A dose deescalation cohort using doses of durvalumab 1500 mg Q4W and monalizumab 7.5 mg Q2W will be implemented if unacceptable toxicity is encountered at the first dose level.

### Example 2: Responses in MSS colorectal cancer patients treated with monalizumab and durvalumab in a CRC dose expansion part of a Phase I clinical study

The dose-expansion part of the phase 1 study aims to recruit patients into four cohorts corresponding to cancers which are not adequately treated with current therapies. One of the cohorts included recurrent or metastatic MSS-CRC subjects. In the MSS-CRC cohort, all subjects needed to have a documented mutation test during screening and confirmed tumor locations from disease assessment for enrolment, and CRC cancers must not have a defective DNA mismatch repair (microsatellite instability), as documented by testing.

Defective DNA mismatch repair was defined by either: (i) High-frequency microsatellite instability with changes detected in 2 or more panels of microsatellite markers (BAT-25, BAT-26, NR-21, NR-24, or MONO-27), or (ii) Immunohistochemical analysis demonstrating absence of protein expression of any one or more of the following proteins: MLH1, MSH2, MSH6, or PMS2.

Subjects cohorts received durvalumab (1500 mg every 4 weeks (Q4W)) in combination with monalizumab at 750 mg every 2 weeks (Q2W).

Results showed that in in the MSS-CRC expansion cohort (58% of patients having received at least 3 lines of prior therapy, n=37 evaluable for efficacy), there were 3 confirmed Partial Response (PR) (including one patient whose response has improved from Partial Response to unconfirmed Complete Response) and 11 Stable Disease (SD), including 3 patients with tumor reduction who continued therapy for >200 days. The Disease Control Rate (DCR) at 16 weeks was 24%.

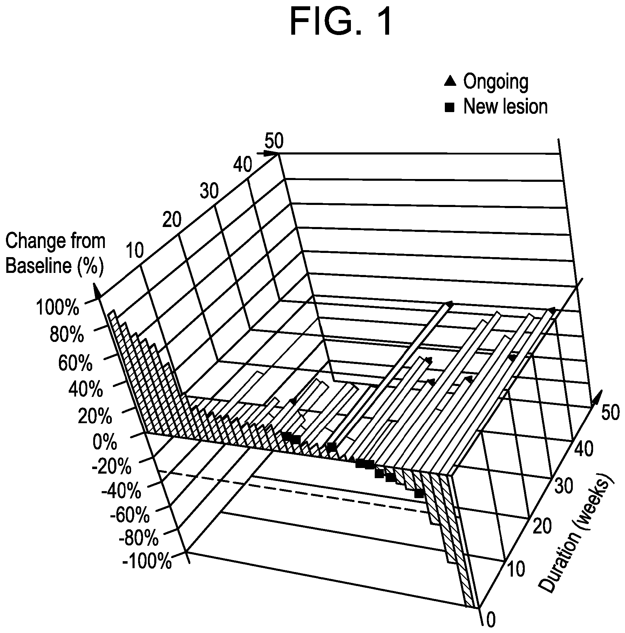

Percent change in tumor size from baseline and duration of treatment in MSS-CRC expansion cohort are represented in Figure 1.

An update of the dose expansion part of the Phase I clinical study on monalizumab and durvalumab in CRC described above shows the results that follow.

Subjects cohorts received durvalumab (1500 mg every 4 weeks (Q4W)) in combination with monalizumab at 750 mg every 2 weeks (Q2W).

Results showed that in in the MSS-CRC expansion cohort (60% of patients having received at least 3 lines of prior therapy, n=39 evaluable for efficacy), there were 1 confirmed complete response, 2 confirmed Partial Response (PR) and 11 Stable Disease (SD) (Tables 1 and 2).

**Table 1. Prior anti-cancer treatment - as treated population**

| | | MSS-CRC |
|---|---|---|
| | | N = 40 |
| Number of prior regimens | | |
| | n | 40 |
| | Mean | 3.7 |
| | SD | 1.9 |
| | Median | 3.0 |
| | (Min, Max) | (1, 11) |
| Prior systemic therapy | | 40 (100%) |
| Prior radiation | | 13 (32.5%) |
| Prior surgery | | 32 (80.0%) |
| Prior bone marrow/stem cell transplant | | 0 |
| Most recent line of therapy for recurrent/metastatic disease | | |
| | n | 40 |
| | First line | 7 (17.5%) |
| | Second line | 9 (22.5%) |
| | Third line or greater | 24 (60%) |
| | NA | 0 |

**Table 2. Disease response in response evaluable population**

| | | MSS-CRC |
|---|---|---|
| | | N = 39 |
| Best Overall Response | | |
| | CR | 1 (2.6%) |
| | PR | 2 (5.1%) |
| | SD | 11 (28.2%) |
| | Unconfirmed PR | 0 |
| | PD | 22 (56.4%) |
| | Non-evaluable | 1 (2.6%) |
| | Not available | 2 (5.1%) |
| CR + PR (confirmed and unconfirmed) | | 3 (7.7%) |
| | 95% C.I. | (1.7%-21.9%) |
| CR + PR (ORR) | | 3 (7.7%) |
| | 95% C.I. | (1.7%-21.9%) |
| | Time to Response (weeks) | 3 |
| | Median time^{a} | 8.1 |
| | 95% CI of Median Time^{a} | (7.4-24.6) |

| | | |
|---|---|---|
| ^{a} Median time to response, and median duration of response and median duration of disease control assessed via Kaplan-Meier methods. Response evaluable population includes patients in the as-treated population who have at least one post-baseline disease assessment or discontinued due to death or disease progression prior to the first post-baseline disease assessment. | | |

The Disease Control Rate (DCR) at 16 weeks was 31% and 18% at 24 weeks (Table 3).

**Table 3. Disease response in response evaluable population**

| | | MSS-CRC |
|---|---|---|
| | | N = 39 |
| | Min, Max | (7.4, 24.6) |
| Duration of Response (weeks) | | 3 |
| | Median time^{a} | 16.1 |
| | 95% CI of Median Time^{a} | (15.9 - NE) |
| | Min, Max | (15.9 - 56.4+) |
| CR + PR + SD ≥ 16 weeks (DCR16) | | 12 (30.8%) |
| | 95% CI | (17.0%-47.6%) |
| CR + PR + SD ≥ 24 weeks (DCR24) | | 7 (17.9%) |
| | 95% CI | (7.5%-33.5%) |

| | | |
|---|---|---|
| ^{a} Median time to response, and median duration of response and median duration of disease control assessed via Kaplan-Meier methods. Response evaluable population includes patients in the as-treated population who have at least one post-baseline disease assessment or discontinued due to death or disease progression prior to the first post-baseline disease assessment. | | |

The Median OS obtained thus far is encouraging, being of 10.6 months, which compares favourably to Lonsurf/TAS-102 median OS of 5.7 months (Mayer et al, 2015, N. Engl. J. Med. 372:1909-1919) or against regorafenib reported median OS of 6.4 months (Grothey et al, Lancet 2013, 381(9863): 303-312) in a similar population.

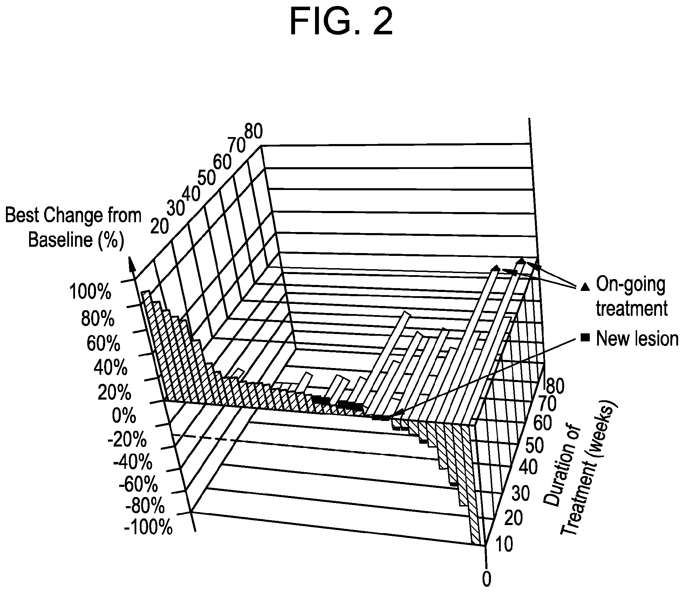

Percent change in tumor size from baseline and duration of treatment in this MSS-CRC expansion cohort are represented in Figure 2, on a longer time period compared to Figure 1.

In conclusion, dose escalation of this first-in-human combination of monalizumab plus durvalumab has been completed, demonstrating a manageable toxicity profile. The data indicate that the monalizumab plus durvalumab combination could bring an improved benefit to patients with MSS-CRC, a population historically nonresponsive to PD-1/PD-L1 blockade.

Unless otherwise stated, all exact values provided herein are representative of corresponding approximate values (e.g., all exact exemplary values provided with respect to a particular factor or measurement can be considered to also provide a corresponding approximate measurement, modified by "about," where appropriate). Where "about" is used in connection with a number, this can be specified as including values corresponding to +/-10% of the specified number.

The description herein of any aspect or embodiment of the invention using terms such as "comprising", "having," "including," or "containing" with reference to an element or elements is intended to provide support for a similar aspect or embodiment of the invention that "consists of", "consists essentially of", or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context).

The use of any and all examples, or exemplary language (e.g., "such as") provided herein, is intended merely to better illuminate the invention and does not pose a limitation on the scope of the invention, which is defined by the appended claims. No language in the specification should be construed as indicating any non-claimed element as essential to the practice of the invention.

## Claims

1. A NKG2A neutralizing agent for use in a method of treating a cancer and/or eliciting an anti-tumor immune response in an individual in need thereof, wherein said individual has a tumor that is not MSI-High (MSI-H) and/or not DNA mismatch repair (MMR) defective, the method comprising administering to said individual a therapeutically effective amount of the NKG2A neutralizing agent and a therapeutically effective amount of a PD-1 neutralizing agent, wherein said NKG2A neutralizing agent is an antibody that inhibits binding of NKG2A to HLA-E.

2. A PD-1 neutralizing agent for use in a method of treating a cancer and/or eliciting an antitumor immune response in an individual in need thereof, wherein said individual has a tumor that is not MSI-High (MSI-H) and/or not DNA mismatch repair (MMR) defective, the method comprising administering to said individual a therapeutically effective amount of the PD-1 neutralizing agent and a therapeutically effective amount of a NKG2A neutralizing agent, wherein said NKG2A neutralizing agent is an antibody that inhibits binding of NKG2A to HLA-E.

3. The NKG2A neutralizing agent for the use of claim 1 or PD-1 neutralizing agent for the use of claim 2, wherein said individual has a tumor that does not have microsatellite instability detected in two or more microsatellite markers, optionally wherein said individual has a tumor that has no alteration detected in two or more of the microsatellite markers selected from the group consisting of BAT-25, BAT-26, NR-21, NR-24, and MONO27.

4. The NKG2A neutralizing agent for the use of claim 1 or 3 or PD-1 neutralizing agent for the use of claim 2 or 3, wherein said individual has a tumor that does not have an alteration in expression of a DNA mismatch repair (MMR) protein, optionally wherein said individual has a tumor that does not have decreased or absence of expression of at least one MMR protein selected from MSH2, MLH1, MSH6 and PMS2, and/or said individual has a tumor that is microsatellite stable (MSS).

5. The NKG2A neutralizing agent for the use of any of claims 1 to 4 or PD-1 neutralizing agent for the use of any of claims 2 to 4, wherein said individual has a cancer selected from the group consisting of colorectal cancer, colon cancer and rectal cancer, optionally wherein said individual has a colorectal cancer, optionally an advanced recurrent or a metastatic colorectal cancer or wherein said individual has a MSS-colorectal cancer (MSS-CRC).

6. The NKG2A neutralizing agent for the use of any of claims 1 to 5 or PD-1 neutralizing agent for the use of any of claims 2 to 5, wherein said method comprises:
a) a preliminary step of determining whether said individual has a tumor that is not MSI-H and/or DNA mismatch repair defective, optionally determining whether said individual has a tumor that does not have microsatellite instability detected in two or more microsatellite markers, optionally wherein said individual has a tumor that has no alteration detected in two or more of the microsatellite markers selected from the group consisting of BAT-25, BAT-26, NR-21, NR-24, and MONO27; and/or
b) a preliminary step of determining whether said individual has a tumor that does not have an alteration in expression of a DNA mismatch repair (MMR) protein, optionally whether said individual has a tumor that does not have decreased or absence of expression of at least one MMR protein selected from MSH2, MLH1, MSH6, and PMS2.

7. The NKG2A neutralizing agent for the use of any of claims 1 to 6 or PD-1 neutralizing agent for the use of any of claims 2 to 6, wherein said NKG2A neutralizing agent is an antibody that binds a human NKG2A protein, optionally a humanized or human anti-NKG2A antibody.

8. The NKG2A neutralizing agent for the use of any of claims 1 to 7 or PD-1 neutralizing agent for the use of any of claims 2 to 7, optionally wherein said NKG2A neutralizing agent comprises the heavy chain H-CDR1, H-CDR2 and H-CDR3 domains having the sequences of SEQ ID NOS: 16-18, and the light chain L-CDR1, L-CDR2 and L-CDR3 domains having the sequences of SEQ ID NOS: 19-21, respectively, optionally wherein said NKG2A neutralizing agent is monalizumab.

9. The NKG2A neutralizing agent for the use of any of claims 1 to 8 or PD-1 neutralizing agent for the use of any of claims 2 to 8, wherein said PD-1 neutralizing agent is an antibody, optionally wherein said PD-1 neutralizing agent is an antibody that binds a human PD-1 polypeptide, optionally said PD-1 neutralizing agent is a human anti-PD-1 antibody.

10. The NKG2A neutralizing agent for the use of any of claims 1 to 8 or PD-1 neutralizing agent for the use of any of claims 2 to 8, wherein said PD-1 neutralizing agent is an antibody that binds a human PD-L1 polypeptide, optionally said PD-1 neutralizing agent is a human anti-PD-L1 antibody.

11. The NKG2A neutralizing agent for the use of any of claims 1 to 8 or PD-1 neutralizing agent for the use of any of claims 2 to 8, wherein said PD-1 neutralizing agent comprises the heavy chain H-CDR1, H-CDR2 and H-CDR3 domains having the amino acid sequences of SEQ ID NOS: 28-30, and the light chain L-CDR1, L-CDR2 and L-CDR3 domains having the amino acid sequences of SEQ ID NOS: 31-33, respectively.

12. The NKG2A neutralizing agent for the use of any of claims 1 to 8 or PD-1 neutralizing agent for the use of any of claims 2 to 9, wherein said PD-1 neutralizing agent is durvalumab.

13. The NKG2A neutralizing agent for the use of any of claims 1 to 8 or PD-1 neutralizing agent for the use of any of claims 2 to 8, wherein said NKG2A neutralizing agent is monalizumab and said PD-1 neutralizing agent is durvalumab.

14. The NKG2A neutralizing agent for the use of any of claims 1 to 13 or PD-1 neutralizing agent for the use of any of claims 2 to 13, wherein said NKG2A neutralizing agent and said PD-1 neutralizing agent are administered simultaneously, separately, or sequentially and/or wherein said NKG2A neutralizing agent and said PD-1 neutralizing agent are formulated for separate administration and are administered concurrently or sequentially, and/or wherein said NKG2A neutralizing agent is administered at a dose ranging from 0.1 to 10 mg/kg and said PD-1 neutralizing agent is administered at a dose ranging from 1 to 20 mg/kg, optionally said NKG2A neutralizing agent is administered at a dose of 10 mg/kg and said PD-1 neutralizing agent is administered at a dose of 20 mg/kg, optionally said NKG2A neutralizing agent is monalizumab administered at a fixed dose of 750 mg every 2 weeks and said PD-1 neutralizing agent is durvalumab administered at a fixed dose of 1500 mg every 4 weeks.

15. A kit for use in the treatment of a MSS-CRC in a patient in need thereof, wherein:
i. the kit comprises a pharmaceutical composition containing a NKG2A neutralizing agent such as an anti-NKG2A antibody, and a PD-1 neutralizing agent such as an anti-PD-1 antibody or an anti-PD-L1 antibody; or
ii. the kit comprises a first pharmaceutical composition containing a PD-1 neutralizing agent such as an anti-PD-1 antibody or an anti-PD-L1 antibody, and a second pharmaceutical composition containing a NKG2A neutralizing agent such as an anti-NKG2A antibody; or
iii. the kit comprises a pharmaceutical composition containing a NKG2A neutralizing agent such as an anti-NKG2A antibody, and the treatment comprises administering to said patient a therapeutically effective amount of the NKG2A neutralizing agent and a therapeutically effective amount of a PD-1 neutralizing agent such as an anti-PD-1 antibody or an anti-PD-L1 antibody; or
iv. the kit comprises a pharmaceutical composition containing a PD-1 neutralizing agent such as an anti-PD-1 antibody or an anti-PD-L1 antibody, and the treatment comprises administering to said patient a therapeutically effective amount of the PD-1 neutralizing agent and a therapeutically effective amount of a NKG2A neutralizing agent,
wherein said NKG2A neutralizing agent is an antibody that inhibits binding of NKG2A to HLA-E, optionally wherein said NKG2A neutralizing agent is monalizumab and said PD-1 neutralizing agent is durvalumab.

## Patentansprüche

1. NKG2A-neutralisierendes Mittel zur Verwendung in einem Verfahren zum Behandeln von Krebs und/oder Hervorrufen einer Anti-Tumor-Immunantwort bei einem Individuum, das dessen bedarf, wobei das Individuum einen Tumor aufweist, der nicht MSI-High (MSI-H) und/oder nicht DNA-Fehlpaarungsreparatur(MMR)-defekt ist, wobei das Verfahren Verabreichen einer therapeutisch wirksamen Menge des NKG2A-neutralisierenden Mittels und einer therapeutisch wirksamen Menge eines PD-1-neutralisierenden Mittels an das Individuum umfasst, wobei das NKG2A-neutralisierende Mittel ein Antikörper ist, der die Bindung von NKG2A an HLA-E hemmt.

2. PD-1-neutralisierendes Mittel zur Verwendung in einem Verfahren zum Behandeln von Krebs und/oder Hervorrufen einer Anti-Tumor-Immunantwort bei einem Individuum, das dessen bedarf, wobei das Individuum einen Tumor aufweist, der nicht MSI-High (MSI-H) und/oder nicht DNA-Fehlpaarungsreparatur(MMR)-defekt ist, wobei das Verfahren Verabreichen einer therapeutisch wirksamen Menge des PD-1-neutralisierenden Mittels und einer therapeutisch wirksamen Menge eines NKG2A-neutralisierenden Mittels an das Individuum umfasst, wobei das NKG2A-neutralisierende Mittel ein Antikörper ist, der die Bindung von NKG2A an HLA-E hemmt.

3. NKG2A-neutralisierendes Mittel zur Verwendung nach Anspruch 1 oder PD-1-neutralisierendes Mittel zur Verwendung nach Anspruch 2, wobei das Individuum einen Tumor aufweist, der keine Mikrosatelliteninstabilität aufweist, die in zwei oder mehr Mikrosatellitenmarkern detektiert wurde, wobei das Individuum optional einen Tumor aufweist, der keine Veränderung aufweist, die in zwei oder mehr der Mikrosatellitenmarker detektiert wurde, ausgewählt aus der Gruppe bestehend aus BAT-25, BAT-26, NR-21, NR-24 und MONO27.

4. NKG2A-neutralisierendes Mittel zur Verwendung nach Anspruch 1 oder 3 oder PD-1-neutralisierendes Mittel zur Verwendung nach Anspruch 2 oder 3, wobei das Individuum einen Tumor aufweist, der keine Veränderung der Expression eines DNA-Fehlpaarungsreparatur(MMR)-Proteins aufweist, wobei das Individuum optional einen Tumor aufweist, der keine verringerte oder fehlende Expression von mindestens einem MMR-Protein aufweist, ausgewählt aus MSH2, MLH1, MSH6 und PMS2, und/oder das Individuum einen Tumor aufweist, der mikrosatellitenstabil (MSS) ist.

5. NKG2A-neutralisierendes Mittel zur Verwendung nach einem der Ansprüche 1 bis 4 oder PD-1-neutralisierendes Mittel zur Verwendung nach einem der Ansprüche 2 bis 4, wobei das Individuum einen Krebs aufweist, ausgewählt aus der Gruppe bestehend aus kolorektalem Karzinom, Kolonkarzinom und Rektumkarzinom, wobei das Individuum optional ein kolorektales Karzinom aufweist, optional ein fortgeschrittenes rezidivierendes oder ein metastatisches kolorektales Karzinom, oder wobei das Individuum ein MSS-kolorektales Karzinom (MSS-CRC) aufweist.

6. NKG2A-neutralisierendes Mittel zur Verwendung nach einem der Ansprüche 1 bis 5 oder PD-1-neutralisierendes Mittel zur Verwendung nach einem der Ansprüche 2 bis 5, wobei das Verfahren umfasst:
a) einen vorläufigen Schritt des Bestimmens, ob das Individuum einen Tumor aufweist, der nicht MSI-H und/oder DNA-Fehlpaarungsreparatur-defekt ist, optional Bestimmen, ob das Individuum einen Tumor aufweist, der keine Mikrosatelliteninstabilität aufweist, die in zwei oder mehr Mikrosatellitenmarkern detektiert wurde, wobei das Individuum optional einen Tumor aufweist, der keine Veränderung aufweist, die in zwei oder mehr der Mikrosatellitenmarker detektiert wurde, ausgewählt aus der Gruppe bestehend aus BAT-25, BAT-26, NR-21, NR-24 und MONO27; und/oder
b) einen vorläufigen Schritt des Bestimmens, ob das Individuum einen Tumor aufweist, der keine Veränderung der Expression eines DNA-Fehlpaarungsreparatur(MMR)-Proteins aufweist, optional ob das Individuum einen Tumor aufweist, der keine verringerte oder fehlende Expression von mindestens einem MMR-Protein aufweist, ausgewählt aus MSH2, MLH1, MSH6 und PMS2.

7. NKG2A-neutralisierendes Mittel zur Verwendung nach einem der Ansprüche 1 bis 6 oder PD-1-neutralisierendes Mittel zur Verwendung nach einem der Ansprüche 2 bis 6, wobei das NKG2A-neutralisierende Mittel ein Antikörper ist, der ein humanes NKG2A-Protein bindet, optional ein humanisierter oder humaner Anti-NKG2A-Antikörper.

8. NKG2A-neutralisierendes Mittel zur Verwendung nach einem der Ansprüche 1 bis 7 oder PD-1-neutralisierendes Mittel zur Verwendung nach einem der Ansprüche 2 bis 7, wobei das NKG2A-neutralisierende Mittel optional die Domänen der schweren Kette H-CDR1, H-CDR2 und H-CDR3 mit den Sequenzen von SEQ ID NOS: 16-18 und die Domänen der leichten Kette L-CDR1, L-CDR2 und L-CDR3 mit den Sequenzen von SEQ ID NOS: 19-21 umfasst, beziehungsweise, wobei das NKG2A-neutralisierende Mittel optional Monalizumab ist.

9. NKG2A-neutralisierendes Mittel zur Verwendung nach einem der Ansprüche 1 bis 8 oder PD-1-neutralisierendes Mittel zur Verwendung nach einem der Ansprüche 2 bis 8, wobei das PD-1-neutralisierende Mittel ein Antikörper ist, wobei das PD-1-neutralisierende Mittel optional ein Antikörper ist, der ein humanes PD-1-Polypeptid bindet, wobei das PD-1-neutralisierende Mittel optional ein humaner Anti-PD-1-Antikörper ist.

10. NKG2A-neutralisierendes Mittel zur Verwendung nach einem der Ansprüche 1 bis 8 oder PD-1-neutralisierendes Mittel zur Verwendung nach einem der Ansprüche 2 bis 8, wobei das PD-1-neutralisierende Mittel ein Antikörper ist, der ein humanes PD-L1-Polypeptid bindet, wobei das PD-1-neutralisierende Mittel optional ein humaner Anti-PD-L1-Antikörper ist.

11. NKG2A-neutralisierendes Mittel zur Verwendung nach einem der Ansprüche 1 bis 8 oder PD-1-neutralisierendes Mittel zur Verwendung nach einem der Ansprüche 2 bis 8, wobei das PD-1-neutralisierende Mittel die Domänen der schweren Kette H-CDR1, H-CDR2 und H-CDR3 mit den Aminosäuresequenzen von SEQ ID NOS: 28-30 und die Domänen der leichten Kette L-CDR1, L-CDR2 und L-CDR3 mit den Aminosäuresequenzen von SEQ ID NOS: 31-33 umfasst, beziehungsweise.

12. NKG2A-neutralisierendes Mittel zur Verwendung nach einem der Ansprüche 1 bis 8 oder PD-1-neutralisierendes Mittel zur Verwendung nach einem der Ansprüche 2 bis 9, wobei das PD-1-neutralisierende Mittel Durvalumab ist.

13. NKG2A-neutralisierendes Mittel zur Verwendung nach einem der Ansprüche 1 bis 8 oder PD-1-neutralisierendes Mittel zur Verwendung nach einem der Ansprüche 2 bis 8, wobei das NKG2A-neutralisierende Mittel Monalizumab ist und das PD-1-neutralisierende Mittel Durvalumab ist.

14. NKG2A-neutralisierendes Mittel zur Verwendung nach einem der Ansprüche 1 bis 13 oder PD-1-neutralisierendes Mittel zur Verwendung nach einem der Ansprüche 2 bis 13, wobei das NKG2A-neutralisierende Mittel und das PD-1-neutralisierende Mittel gleichzeitig, getrennt oder sequenziell verabreicht werden und/oder wobei das NKG2A-neutralisierende Mittel und das PD-1-neutralisierende Mittel zur getrennten Verabreichung formuliert sind und gleichzeitig oder sequenziell verabreicht werden, und/oder wobei das NKG2A-neutralisierende Mittel in einer Dosis im Bereich von 0,1 bis 10 mg/kg verabreicht wird und das PD-1-neutralisierende Mittel in einer Dosis im Bereich von 1 bis 20 mg/kg verabreicht wird, wobei das NKG2A-neutralisierende Mittel optional in einer Dosis von 10 mg/kg verabreicht wird und das PD-1-neutralisierende Mittel in einer Dosis von 20 mg/kg verabreicht wird, wobei das NKG2A-neutralisierende Mittel optional Monalizumab ist, das in einer Festdosis von 750 mg alle 2 Wochen verabreicht wird, und das PD-1-neutralisierende Mittel Durvalumab ist, das in einer Festdosis von 1500 mg alle 4 Wochen verabreicht wird.

15. Kit zur Verwendung bei der Behandlung eines MSS-kolorektalen Karzinoms (MSS-CRC) bei einem Patienten, der dessen bedarf, wobei:
i. der Kit eine pharmazeutische Zusammensetzung umfasst, die ein NKG2A-neutralisierendes Mittel wie einen Anti-NKG2A-Antikörper und ein PD-1-neutralisierendes Mittel wie einen Anti-PD-1-Antikörper oder einen Anti-PD-L1-Antikörper enthält; oder
ii. der Kit eine erste pharmazeutische Zusammensetzung umfasst, die ein PD-1-neutralisierendes Mittel wie einen Anti-PD-1-Antikörper oder einen Anti-PD-L1-Antikörper enthält, und eine zweite pharmazeutische Zusammensetzung, die ein NKG2A-neutralisierendes Mittel wie einen Anti-NKG2A-Antikörper enthält; oder
iii. der Kit eine pharmazeutische Zusammensetzung umfasst, die ein NKG2A-neutralisierendes Mittel wie einen Anti-NKG2A-Antikörper enthält, und die Behandlung Verabreichen einer therapeutisch wirksamen Menge des NKG2A-neutralisierenden Mittels und einer therapeutisch wirksamen Menge eines PD-1-neutralisierenden Mittels wie eines Anti-PD-1-Antikörpers oder eines Anti-PD-L1-Antikörpers an den Patienten umfasst; oder
iv. der Kit eine pharmazeutische Zusammensetzung umfasst, die ein PD-1-neutralisierendes Mittel wie einen Anti-PD-1-Antikörper oder einen Anti-PD-L1-Antikörper enthält, und die Behandlung Verabreichen einer therapeutisch wirksamen Menge des PD-1-neutralisierenden Mittels und einer therapeutisch wirksamen Menge eines NKG2A-neutralisierenden Mittels an den Patienten umfasst, wobei das NKG2A-neutralisierende Mittel ein Antikörper ist, der die Bindung von NKG2A an HLA-E hemmt, wobei das NKG2A-neutralisierende Mittel optional Monalizumab ist und das PD-1-neutralisierende Mittel Durvalumab ist.

## Revendications

1. Agent neutralisant NKG2A pour utilisation dans un procédé de traitement d'un cancer et/ou d'élicitation d'une réponse immunitaire anti-tumorale chez un individu qui en a besoin, dans lequel ledit individu présente une tumeur qui n'est pas MSI-élevée (MSI-H) et/ou qui n'est pas déficiente en réparation des mésappariements de l'ADN, le procédé comprenant l'administration audit individu d'une quantité thérapeutiquement efficace de l'agent neutralisant NKG2A et d'une quantité thérapeutiquement efficace d'un agent neutralisant PD-1, dans lequel ledit agent neutralisant NKG2A est un anticorps qui inhibe la liaison de NKG2A à HLA-E.

2. Agent neutralisant PD-1 pour utilisation dans un procédé de traitement d'un cancer et/ou d'élicitation d'une réponse immunitaire anti-tumorale chez un individu qui en a besoin, dans lequel ledit individu présente une tumeur qui n'est pas MSI-élevée (MSI-H) et/ou qui n'est pas déficiente en réparation des mésappariements de l'ADN, le procédé comprenant l'administration audit individu d'une quantité thérapeutiquement efficace de l'agent neutralisant PD-1 et d'une quantité thérapeutiquement efficace d'un agent neutralisant NKG2A, dans lequel ledit agent neutralisant NKG2A est un anticorps qui inhibe la liaison de NKG2A à HLA-E.

3. Agent neutralisant NKG2A pour l'utilisation selon la revendication 1 ou agent neutralisant PD-1 pour l'utilisation selon la revendication 2, dans lequel ledit individu présente une tumeur qui ne présente pas d'instabilité des microsatellites détectée dans deux marqueurs microsatellites ou plus, optionnellement dans lequel ledit individu présente une tumeur qui ne présente aucune altération détectée dans deux ou plus des marqueurs microsatellites sélectionnés dans le groupe consistant en BAT-25, BAT-26, NR-21, NR-24 et MONO27.

4. Agent neutralisant NKG2A pour l'utilisation selon la revendication 1 ou 3 ou agent neutralisant PD-1 pour l'utilisation selon la revendication 2 ou 3, dans lequel ledit individu présente une tumeur qui ne présente pas d'altération dans l'expression d'une protéine MMR de réparation des mésappariements de l'ADN, optionnellement dans lequel ledit individu présente une tumeur qui ne présente pas de diminution ou absence d'expression d'au moins une protéine MMR sélectionnée parmi MSH2, MLH1, MSH6 et PMS2, et/ou ledit individu présente une tumeur qui est stable sur le plan des microsatellites (MSS).

5. Agent neutralisant NKG2A pour l'utilisation selon l'une quelconque des revendications 1 à 4 ou agent neutralisant PD-1 pour l'utilisation selon l'une quelconque des revendications 2 à 4, dans lequel ledit individu présente un cancer sélectionné dans le groupe consistant en cancer colorectal, cancer du côlon et cancer rectal, optionnellement dans lequel ledit individu présente un cancer colorectal, optionnellement un cancer colorectal récurrent avancé ou métastatique ou dans lequel ledit individu présente un cancer colorectal MSS (MSS-CRC).

6. Agent neutralisant NKG2A pour l'utilisation selon l'une quelconque des revendications 1 à 5 ou agent neutralisant PD-1 pour l'utilisation selon l'une quelconque des revendications 2 à 5, dans lequel ledit procédé comprend :
a) une étape préliminaire consistant à déterminer si ledit individu présente une tumeur qui n'est pas MSI-H et/ou déficiente en réparation des mésappariements de l'ADN, optionnellement à déterminer si ledit individu présente une tumeur qui ne présente pas d'instabilité des microsatellites détectée dans deux marqueurs microsatellites ou plus, optionnellement dans lequel ledit individu présente une tumeur qui ne présente aucune altération détectée dans deux ou plus des marqueurs microsatellites sélectionnés dans le groupe consistant en BAT-25, BAT-26, NR-21, NR-24 et MONO27 ; et/ou
b) une étape préliminaire consistant à déterminer si ledit individu présente une tumeur qui ne présente pas d'altération dans l'expression d'une protéine MMR de réparation des mésappariements de l'ADN, optionnellement si ledit individu présente une tumeur qui ne présente pas de diminution ou absence d'expression d'au moins une protéine MMR sélectionnée parmi MSH2, MLH1, MSH6 et PMS2.

7. Agent neutralisant NKG2A pour l'utilisation selon l'une quelconque des revendications 1 à 6 ou agent neutralisant PD-1 pour l'utilisation selon l'une quelconque des revendications 2 à 6, dans lequel ledit agent neutralisant NKG2A est un anticorps qui se lie à une protéine NKG2A humaine, optionnellement un anticorps anti-NKG2A humanisé ou humain.

8. Agent neutralisant NKG2A pour l'utilisation selon l'une quelconque des revendications 1 à 7 ou agent neutralisant PD-1 pour l'utilisation selon l'une quelconque des revendications 2 à 7, optionnellement dans lequel ledit agent neutralisant NKG2A comprend les domaines H-CDR1, H-CDR2 et H-CDR3 de la chaîne lourde ayant les séquences de SEQ ID NO : 16-18, et les domaines L-CDR1, L-CDR2 et L-CDR3 de la chaîne légère ayant les séquences de SEQ ID NO : 19-21, respectivement, optionnellement dans lequel ledit agent neutralisant NKG2A est le monalizumab.

9. Agent neutralisant NKG2A pour l'utilisation selon l'une quelconque des revendications 1 à 8 ou agent neutralisant PD-1 pour l'utilisation selon l'une quelconque des revendications 2 à 8, dans lequel ledit agent neutralisant PD-1 est un anticorps, optionnellement dans lequel ledit agent neutralisant PD-1 est un anticorps qui se lie à un polypeptide PD-1 humain, optionnellement ledit agent neutralisant PD-1 est un anticorps anti-PD-1 humain.

10. Agent neutralisant NKG2A pour l'utilisation selon l'une quelconque des revendications 1 à 8 ou agent neutralisant PD-1 pour l'utilisation selon l'une quelconque des revendications 2 à 8, dans lequel ledit agent neutralisant PD-1 est un anticorps qui se lie à un polypeptide PD-L1 humain, optionnellement ledit agent neutralisant PD-1 est un anticorps anti-PD-L1 humain.

11. Agent neutralisant NKG2A pour l'utilisation selon l'une quelconque des revendications 1 à 8 ou agent neutralisant PD-1 pour l'utilisation selon l'une quelconque des revendications 2 à 8, dans lequel ledit agent neutralisant PD-1 comprend les domaines H-CDR1, H-CDR2 et H-CDR3 de la chaîne lourde ayant les séquences d'acides aminés de SEQ ID NO : 28-30, et les domaines L-CDR1, L-CDR2 et L-CDR3 de la chaîne légère ayant les séquences d'acides aminés de SEQ ID NO : 31-33, respectivement.

12. Agent neutralisant NKG2A pour l'utilisation selon l'une quelconque des revendications 1 à 8 ou agent neutralisant PD-1 pour l'utilisation selon l'une quelconque des revendications 2 à 9, dans lequel ledit agent neutralisant PD-1 est le durvalumab.

13. Agent neutralisant NKG2A pour l'utilisation selon l'une quelconque des revendications 1 à 8 ou agent neutralisant PD-1 pour l'utilisation selon l'une quelconque des revendications 2 à 8, dans lequel ledit agent neutralisant NKG2A est le monalizumab et ledit agent neutralisant PD-1 est le durvalumab.

14. Agent neutralisant NKG2A pour l'utilisation selon l'une quelconque des revendications 1 à 13 ou agent neutralisant PD-1 pour l'utilisation selon l'une quelconque des revendications 2 à 13, dans lequel ledit agent neutralisant NKG2A et ledit agent neutralisant PD-1 sont administrés simultanément, séparément, ou séquentiellement et/ou dans lequel ledit agent neutralisant NKG2A et ledit agent neutralisant PD-1 sont formulés pour une administration séparée et sont administrés de manière concomitante ou séquentiellement, et/ou dans lequel ledit agent neutralisant NKG2A est administré à une dose allant de 0,1 à 10 mg/kg et ledit agent neutralisant PD-1 est administré à une dose allant de 1 à 20 mg/kg, optionnellement ledit agent neutralisant NKG2A est administré à une dose de 10 mg/kg et ledit agent neutralisant PD-1 est administré à une dose de 20 mg/kg, optionnellement ledit agent neutralisant NKG2A est le monalizumab administré à une dose fixe de 750 mg toutes les 2 semaines et ledit agent neutralisant PD-1 est le durvalumab administré à une dose fixe de 1500 mg toutes les 4 semaines.

15. Kit pour utilisation dans le traitement d'un MSS-CRC chez un patient qui en a besoin, dans lequel :
i. le kit comprend une composition pharmaceutique contenant un agent neutralisant NKG2A tel qu'un anticorps anti-NKG2A, et un agent neutralisant PD-1 tel qu'un anticorps anti-PD-1 ou un anticorps anti-PD-L1 ; ou
ii. le kit comprend une première composition pharmaceutique contenant un agent neutralisant PD-1 tel qu'un anticorps anti-PD-1 ou un anticorps anti-PD-L1, et une deuxième composition pharmaceutique contenant un agent neutralisant NKG2A tel qu'un anticorps anti-NKG2A ; ou
iii. le kit comprend une composition pharmaceutique contenant un agent neutralisant NKG2A tel qu'un anticorps anti-NKG2A, et le traitement comprend l'administration audit patient d'une quantité thérapeutiquement efficace de l'agent neutralisant NKG2A et d'une quantité thérapeutiquement efficace d'un agent neutralisant PD-1 tel qu'un anticorps anti-PD-1 ou un anticorps anti-PD-L1 ; ou
iv. le kit comprend une composition pharmaceutique contenant un agent neutralisant PD-1 tel qu'un anticorps anti-PD-1 ou un anticorps anti-PD-L1, et le traitement comprend l'administration audit patient d'une quantité thérapeutiquement efficace de l'agent neutralisant PD-1 et d'une quantité thérapeutiquement efficace d'un agent neutralisant NKG2A,
dans lequel ledit agent neutralisant NKG2A est un anticorps qui inhibe la liaison de NKG2A à HLA-E, optionnellement dans lequel ledit agent neutralisant NKG2A est le monalizumab et ledit agent neutralisant PD-1 est le durvalumab.
